# EUROPEAN PATENT APPLICATION

(11) **EP 4 560 318 A1**
(43) Date of publication of application: **28.05.2025**
(21) Application number: 24842930.0
(22) Date of filing: 01.07.2024
(51) Int. Cl.: G01N 33/573

(54) **TRACP-5B MEASUREMENT METHOD AND KIT THEREFOR**

(30) Priority: 18.07.2023 JP 2023116935
(71) Applicant: Nitto Boseki Co., Ltd., Fukushima-shi, Fukushima 960-8161 (JP)
(72) Inventor: HONDA Toru, Koriyama-shi, Fukushima 963-8061 (JP); IMAI Hiroko, Koriyama-shi, Fukushima 963-8061 (JP); KAWASAKI Daisuke, Koriyama-shi, Fukushima 963-8061 (JP); KIKUCHI Wataru, Koriyama-shi, Fukushima 963-8061 (JP); SASAGAWA Kumiko, Koriyama-shi, Fukushima 963-8061 (JP); ITO Kazue, Koriyama-shi, Fukushima 963-8061 (JP)
(74) Representative: Hoffmann Eitle
(86) International application number: PCT/JP2024/023774
(87) International publication number: WO 2025/018136

(57) **Abstract**

In measurement of TRACP-5b, there is provided a measurement method capable of selectively detecting TRACP-5b by further reducing the influence of coexisting TRACP-5a.

A reaction for detecting TRACP-5b is performed in the presence of an organic sulfonic acid, sulfate ester, or salt thereof, wherein the organic sulfonic acid, sulfate ester, and/or salt has a sulfo group (-S(O)₂OH), sulfuric acid group (-OS(O)₂OH), and/or salt thereof at a density of 6.5 mmol/g or more.

## Description

### TECHNICAL FIELD

The present invention relates to a method for measuring TRACP-5b and a kit therefor, and more specifically, to a method for selectively measuring TRACP-5b without any influence of TRACP-5a and a kit therefor.

### BACKGROUND ART

An acid phosphatase (ACP) is an enzyme having an activity of hydrolyzing an organic monophosphate under acidic conditions. ACP has multiple isozymes and is distributed in a variety of tissues. ACP present in serum is known to be derived from platelet, erythrocyte, prostate, osteoclast, and macrophage. These isozymes are separated with polyacrylamide gel electrophoresis, in which osteoclast-derived and macrophage-derived ACPs are located at 5th from the origin. The osteoclast-derived and macrophage-derived ACPs have resistance to tartaric acid and are also called Band-5 Tartrate-resistant Acid Phosphatase (TRACP-5) (Patent Literature 5, Lam W. K. W. et al., Clin. Chem. 24(7): 1105-1108, 1978 and Lam W. K. W. et al., Clin. Biochem. 14, 177-181, 1981, etc.). TRACP-5 is classified into TRACP-5a having a large amount of sialic acid binding to glycans and TRACP-5b having a small amount of sialic acid binding to the same, and 5a is derived from macrophages, and 5b is derived from osteoclasts. Since the concentration of TRACP-5b in the serum sensitively reflects the activity of osteoclasts, TRACP-5b is utilized as a bone resorption marker for assisting diagnosis of metabolic bone disease and bone metastatic cancer and determining therapeutic effects on the same. Therefore, a method for selectively measuring TRACP-5b from ACP in serum has been studied.

Conventionally, there are methods for measuring TRACP-5 comprising performing an enzymatic reaction with a synthetic substrate such as p-nitrophenyl phosphate in the presence of tartaric acid, and subjecting the resulting reaction product to colorimetric reaction to determine the enzyme activity. In this method, prostate-derived ACP can be inhibited by tartaric acid, but TRACP-5a, and erythrocyte-derived and platelet-derived ACPs have resistance to tartaric acid. Therefore, these isozymes are also measured, and TRACP-5b cannot be specifically measured in this method.

As improvement of the above method in terms of the specificity of TRACP-5b, known is a method comprising incubating a serum-5 times- diluted solution at 37°C for 1 hour, and then measuring the enzyme activity in the same manner (Non-Patent Literature 3). In this method, the influence of erythrocyte-derived ACP can also be avoided in addition to that of prostate-derived ACP, but the influence of TRACP-5a and platelet-derived ACP cannot still be avoided.

There is another method for specifically measuring the activity of TRACP-5b, in which the activity of TRACP-5b is calculated by subtracting ACP activity measured in the presence of tartaric acid and fluoride from ACP activity measured in the presence of tartaric acid (Patent Literature 1). In this method, differences in the sensitivity to fluoride can be utilized to avoid the influence of erythrocyte-derived and platelet-derived ACPs. However, the influence of TRACP-5a cannot be removed, and the activity of TRACP-5b obtained by the subtraction has a problem of insufficient accuracy.

Still another method has been reported, in which the activity of TRACP-5b is specifically measured using acidic mucopolysaccharide as an inhibitor of acid phosphatase other than TRACP-5b (Patent Literature 2). However, the inhibitory effect of the acidic mucopolysaccharide on the TRACP-5a activity is not sufficient, and it is required to further improve the specificity for TRACP-5b.

A method for measuring the activity of TRACP-5b using a chemiluminescent substrate is known, wherein the method comprises reacting TRACP-5b with a chemiluminescent 1,2-dioxetane-based compound under an acidic condition, and then measuring the luminescence intensity of the reaction solution under an alkaline condition (Patent Literature 3). In addition, a method for specifically measuring TRACP-5b has been reported, in which the measurement is performed by utilizing increase in the emission intensity ratio of TRACP-5b to TRAPC-5a when a specific buffer such as MES is used or when manganese ions or cobalt ions are added during the enzymatic reaction (Patent Literature 4). However, these methods are limited to activity measurement using a chemiluminescent substrate, and there are needs for a less expensive method for specifically measuring TRACP-5b.

There is an immunoassay method for TRACP-5b using an antibody, wherein the method comprises binding both TRACP-5a and TRACP-5b to an antibody specific to TRACP-5, and then specifically measuring TRACP-5b utilizing the difference in optimal pH between TRACP-5a and TRACP-5b (Patent Literature 5). However, only the difference in optimal pH is insufficient in specificity for TRACP-5b. In addition, the amount of TRACP-5b is obtained by subtraction and therefore also has a problem of insufficient accuracy.

There is also a method for more specifically measuring TRACP-5b, in which an antibody having a certain selectivity for TRACP-5b and 2-chloro-4-nitrophenyl phosphate as a chromogenic substrate are used to measure the activity (Patent Literatures 6 and 7). However, this antibody is also desired to further improve the selectivity for TRACP-5b.

There is also a method for quantifying the amount of TRACP-5b protein using two clones of monoclonal antibodies having high specificity for TRACP-5b (Patent Literature 8).

### CITATION LIST

### PATENT LITERATURE

PATENT LITERATURE 1: JP-A-10-337198
PATENT LITERATURE 2: JP-A-2001-231595
PATENT LITERATURE 3: JP-A-2011-024470
PATENT LITERATURE 4: JP-A-2022-174015
PATENT LITERATURE 5: JP-A-2002-510050
PATENT LITERATURE 6: JP-A-2004-026805
PATENT LITERATURE 7: WO2004/077059
PATENT LITERATURE 8: WO2016/027697

### NON-PATENT LITERATURE

NON-PATENT LITERATURE 1: Non-Patent Literature Clin. Chem. 33: 458-462. 1987

### SUMMARY OF INVENTION

### TECHNICAL PROBLEM

An object of the present invention is to provide a measurement method capable of selectively detecting TRACP-5b by further reducing the influence of coexisting TRACP-5a in measurement of TRACP-5b, compared with the above-described conventional techniques.

### SOLUTION TO PROBLEM

The present inventors have studied a measurement method capable of selectively detecting TRACP-5b, and have found that the influence of TRACP-5a can be significantly reduced when in a reaction system in which TRACP-5a may be present, a compound having a sulfo group (-S(O)₂OH), sulfuric acid group (-OS(O)₂OH), and/or salt thereof exists at a certain density or more, whereby TRACP-5b can be selectively detected.

Thus, the present invention provides the following methods for measuring TRACP-5b and kits for measuring TRACP-5b.
[1] A method for measuring tartrate-resistant acid phosphatase 5b (TRACP-5b) in a specimen, the method comprising:
   performing a reaction for detecting TRACP-5b in the presence of an organic sulfonic acid, sulfate ester, or salt thereof, wherein the organic sulfonic acid, sulfate ester, and/or salt has a sulfo group (-S(O)₂OH), sulfuric acid group (-OS(O)₂OH), and/or salt thereof at a density of 6.5 mmol/g or more.
[2] A method for measuring tartrate-resistant acid phosphatase 5b (TRACP-5b) in a specimen, the method being:
   a method for measuring TRACP-5b comprising: performing an enzymatic reaction or an antigen-antibody reaction in the presence of an organic sulfonic acid, sulfate ester, and/or salt thereof, wherein the organic sulfonic acid, sulfate ester, and/or salt has 50 mass% or more of a sulfo group (-S(O)₂OH), sulfuric acid group (-OS(O)₂OH), and/or salt thereof per unit structure in terms of S(O)₂O⁻; or
   the method according to [1], wherein the organic sulfonic acid, sulfate ester, or salt thereof has 50 mass% or more of a sulfo group (-S(O)₂OH), sulfuric acid group (-OS(O)₂OH), and/or salt thereof per unit structure in terms of S(O)₂O⁻.
[3] The method according to [1] or [2], wherein the organic sulfonic acid, sulfate ester, or salt thereof is represented by Formula (1):
   [Chemical Formula 1]

   R-Y-S(O)₂OX··· (1)

   wherein, R is alkyl, alkenyl, or alkynyl having 1 to 3 carbon atoms, Y is a bond or an oxygen atom, and X is hydrogen or an alkali metal; or
   the organic sulfonic acid, sulfate ester, or salt thereof has a repeating unit of Formula (2): 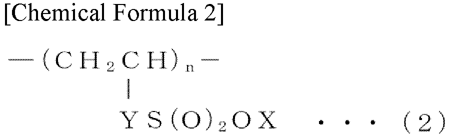
   wherein, Y is a bond or an oxygen atom, n is an integer of 2 to 5000, and X is hydrogen or an alkali metal; or
   the organic sulfonic acid, sulfate ester, or salt thereof has a repeating unit containing a structure of Formula (3): 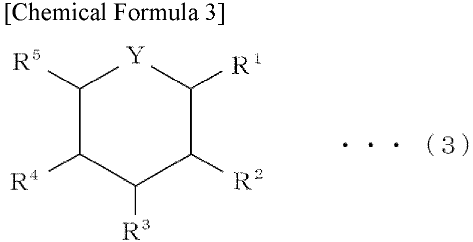
   wherein, Y is O or CH₂, two of R¹ to R⁵ are O- or a bond, and at least two of the rest are O-S(O)₂OX or S(O)₂OX in which X is hydrogen or an alkali metal, and those not corresponding to any of the above are OX or C(O)OX in which X is hydrogen or an alkali metal.
[4] The method according to [1] or [2], wherein the organic sulfonic acid, sulfate ester, or salt thereof is vinyl sulfonic acid or a salt thereof, vinyl sulfate or a salt thereof, polyvinyl sulfonic acid or a salt thereof, polyvinyl sulfuric acid or a salt thereof, methanesulfonic acid or a salt thereof, or dextran sulfate or a salt thereof.
[5] The method according to any one of [1] to [4], wherein the organic sulfonic acid, sulfate ester, or salt thereof is 1) added independently to a specimen or a reaction system, or contained in 2) a specimen diluent, 3) a substrate-containing reagent, or 4) a reagent containing an anti-TRACP-5b antibody (typically an anti-TRACP-5b monoclonal antibody), and thus a reaction for detecting TRACP-5b is performed in the presence of the organic sulfonic acid, sulfate ester, or salt thereof.
[6] The method according to any one of [1] to [5], wherein measurement is performed by an enzyme method, enzyme immunoassay (EIA), enzyme-linked immunosorbent assay (ELISA), chemiluminescent enzyme immunoassay (CLEIA method), chemiluminescent immunoassay (CLIA method), or latex agglutination method.
[7] The method according to any one of [1] to [6], wherein the specimen is blood, serum, or plasma.
[8] A kit for measuring TRACP-5b, comprising:
   an organic sulfonic acid, sulfate ester, and/or salt thereof, wherein the organic sulfonic acid, sulfate ester, and/or salt has a sulfo group (-S(O)₂OH), sulfuric acid group (-OS(O)₂OH), and/or salt thereof at a density of 6.5 mmol/g or more.
[9] A kit for measuring TRACP-5b, being:
   a kit comprising an organic sulfonic acid, sulfate ester, and/or salt thereof, wherein the organic sulfonic acid, sulfate ester, and/or salt has 50 mass% or more of a sulfo group (-S(O)₂OH), sulfuric acid group (-OS(O)₂OH), or salt thereof per unit structure in terms of S(O)₂O⁻; or
   the kit according to [8], wherein the organic sulfonic acid, sulfate ester, and/or salt thereof has 50 mass% or more of a sulfo group (-S(O)₂OH), sulfuric acid group (-OS(O)₂OH), or salt thereof per unit structure in terms of S(O)₂O⁻.
[10] The kit according to [8] or [9], wherein the organic sulfonic acid, sulfate ester, and/or salt thereof is represented by Formula (1):
   [Chemical Formula 4]

   R-Y-S(O)₂OX··· (1)

   wherein, R is alkyl, alkenyl, or alkynyl having 1 to 5 carbon atoms, Y is a bond or an oxygen atom, and X is hydrogen or an alkali metal; or
   the organic sulfonic acid, sulfate ester, and/or salt thereof has a repeating structure of Formula (2): 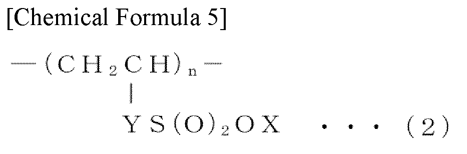
   wherein, Y is a bond or an oxygen atom, n is an integer of 2 to 5000, and X is hydrogen or an alkali metal; or
   the organic sulfonic acid, sulfate ester, and/or salt thereof has a repeating structure containing a structure of Formula (3): 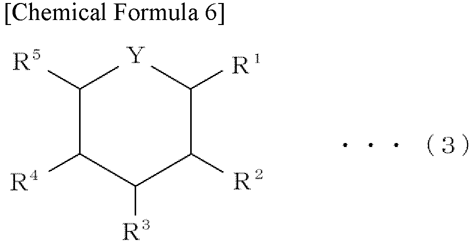
   wherein, Y is O or CH₂, two of R¹ to R⁵ are O- or a bond, and at least two of the rest are O-S(O)₂OX or S(O)₂OX in which X is hydrogen or an alkali metal, and those not corresponding to any of the above are OX or C(O)OX in which X is hydrogen or an alkali metal.
[11] The kit according to [8] or [9], wherein the organic sulfonic acid, sulfate ester, or salt thereof is vinyl sulfate or a salt thereof, vinyl sulfonic acid or a salt thereof, polyvinyl sulfonic acid or a salt thereof, polyvinyl sulfuric acid or a salt thereof, methanesulfonic acid or a salt thereof, or dextran sulfate or a salt thereof.
[12] The kit according to any one of [8] to [11] for measuring TRACP-5b by an enzyme method, the kit comprising:
   a substrate of TRACP-5b; the organic sulfonic acid, sulfate ester, or salt thereof; and optionally a specimen diluent.
[13] The kit according to [12], wherein the organic sulfonic acid, sulfate ester, or salt thereof is contained in a reagent containing the substrate of TRACP-5b or the specimen diluent.
[14] The kit according to any one of [8] to [11] for measuring TRACP-5b by enzyme immunoassay (EIA), the kit comprising:
   a solid support; an anti-TRACP-5b monoclonal antibody that is adsorbed onto the solid support; a substrate of the TRACP-5b; the organic sulfonic acid, sulfate ester, or salt thereof; optionally a specimen diluent; and optionally a wash solution.
[15] The kit according to [14], wherein the organic sulfonic acid, sulfate ester, or salt thereof is contained in a reagent containing the anti-TRACP-5b monoclonal antibody, a reagent containing the substrate of the TRACP-5b, or the specimen diluent.
[16] The kit according to any one of [8] to [11] for measuring TRACP-5b by enzyme-linked immunosorbent assay (ELISA), the kit comprising:
   a solid support; a primary anti-TRACP-5b monoclonal antibody that is adsorbed onto the solid support; a secondary anti-TRACP-5b monoclonal antibody that is capable of binding to TRACP-5b at an epitope different from that of the primary anti-TRACP-5b monoclonal antibody and that is labeled with a substance enabling detection through an enzymatic reaction; a reagent for detecting the substance for labeling through an enzymatic reaction; the organic sulfonic acid, sulfate ester, or salt thereof; optionally a specimen diluent; and optionally a wash solution.
[17] The kit according to [16], wherein the organic sulfonic acid, sulfate ester, or salt thereof is contained in a reagent containing the secondary anti-TRACP-5b monoclonal antibody, the reagent for detecting the substance for labeling through an enzymatic reaction, or the specimen diluent.
[18] The kit according to any one of [8] to [11] for measuring TRACP-5b by chemiluminescent enzyme immunoassay (CLEIA method), the kit comprising:
   a solid support; an anti-TRACP-5b monoclonal antibody that is adsorbed onto the solid support; a chemiluminescent substrate of the TRACP-5b; and the organic sulfonic acid, sulfate ester, or salt thereof; optionally a specimen diluent; and optionally a wash solution.
[19] The kit according to [18], wherein the organic sulfonic acid, sulfate ester, or salt thereof is contained in a reagent containing the chemiluminescent substrate of the TRACP-5b, a reagent containing the anti-TRACP-5b monoclonal antibody, or the specimen diluent.
[20] The kit according to any one of [8] to [11] for measuring TRACP-5b by chemiluminescent immunoassay (CLIA method), the kit comprising:
   a solid support; a primary anti-TRACP-5b monoclonal antibody that is adsorbed onto the solid support; a secondary anti-TRACP-5b monoclonal antibody that is capable of binding to TRACP-5b at an epitope different from that of the primary anti-TRACP-5b monoclonal antibody and that is labeled with a substance enabling detection through chemiluminescence; a reagent for detecting the substance for labeling through chemiluminescence; the organic sulfonic acid, sulfate ester, or salt thereof; optionally a specimen diluent; and optionally a wash solution.
[21] The kit according to [20], wherein the organic sulfonic acid, sulfate ester, or salt thereof is contained in a reagent containing the primary anti-TRACP-5b monoclonal antibody, a reagent containing the secondary anti-TRACP-5b monoclonal antibody, a reagent for detecting the substance for labeling through chemiluminescence, or the specimen diluent.
[22] The kit according to any one of [8] to [11] for measuring TRACP-5b by a latex agglutination method, the kit comprising:
   latex particles; an anti-TRACP-5b antibody adsorbed on the latex particles; the organic sulfonic acid, sulfate ester, or salt thereof; and optionally a specimen diluent.
[23] The kit according to [22], wherein the organic sulfonic acid, sulfate ester, or salt thereof is contained in a reagent containing the anti-TRACP-5b antibody or the specimen diluent.
[24] The kit according to any one of [8] to [23], wherein a specimen to be measured is blood, serum, or plasma.

### ADVANTAGEOUS EFFECTS OF INVENTION

The present application provides a means capable of reducing the influence of tartrate-resistant acid phosphatase 5b (TRACP-5b) and selectively detecting TRACP-5b. The means is therefore useful for diagnosing diseases using TRACP-5b as a marker.

### DESCRIPTION OF EMBODIMENTS

Embodiments of the present invention will be described in detail. However, the present invention should not be understood as being limited to the following embodiments.

An embodiment of the present invention provides a method for measuring tartrate-resistant acid phosphatase 5b (TRACP-5b) in a specimen, wherein the method comprises performing a reaction for detecting TRACP-5b in the presence of a specific organic sulfonic acid, sulfate ester, or salt thereof.

Another embodiment of the present invention also provides a TRACP-5b measurement kit comprising a specific organic sulfonic acid, sulfate ester, or salt thereof.

Hereinafter, these embodiments will be described in detail.

### 1. Method for measuring TRACP-5b

### 1-1. Organic sulfonic acid, sulfate ester, or salt thereof

In this measurement method, an organic sulfonic acid, sulfate ester, or salt thereof having a sulfo group (-S(O)₂OH), sulfuric acid group (-OS(O)₂OH), and/or salt thereof at a density of 6.5 mmol/g or more inhibits TRACP-5a and enables selective measurement of TRACP-5b.

As shown in Examples described later, the inhibitory effect on TRACP-5a increases depending on the density of the sulfo group, sulfuric acid group, or salt thereof. In a preferred embodiment, the organic sulfonic acid, sulfate ester, or salt thereof has the sulfo group, sulfuric acid group, and/or salt thereof at a density of 7.5 mmol/g or more, and more preferably at a density of 8.5 mmol/g or more.

In a preferred embodiment, such an organic sulfonic acid, sulfate ester, or salt thereof has 50 mass% or more, more preferably 60 mass% or more, and still more preferably 70 mass% or more of the sulfo group, sulfuric acid group, and/or salt thereof per unit structure in terms of S(O)₂O⁻.

The term "unit structure" as used herein means, when a compound of interest has a repeating structure, the structure of the repeating unit, and when a compound of interest does not have a repeating structure, the entire structure of the compound. When the unit structure has a salt of the sulfo group (-S(O)₂OH) or the sulfuric acid group (-OS(O)₂OH), the molecular weight of the unit structure is calculated as the sulfo group (-S(O)₂OH) or the sulfuric acid group (-OS(O)₂OH).

One specific embodiment of such an organic sulfonic acid, sulfate ester, or salt thereof includes a compound represented by Formula (1):
[Chemical Formula 7]

R-Y-S(O)₂OX··· (1)

In Formula (1), R is alkyl, alkenyl, or alkynyl having 1 to 3 carbon atoms, preferably alkenyl having 1 to 3 carbon atoms;
Y is a bond or an oxygen atom, preferably a bond; and
X is hydrogen or an alkali metal, preferably hydrogen or Na.

The compound of this embodiment includes methanesulfonic acid or a salt thereof (e.g., Na salt), vinyl sulfonic acid or a salt thereof (e.g., Na salt), or vinyl sulfate or a salt thereof.

Another specific embodiment of the organic sulfonic acid, sulfate ester, or salt thereof includes a compound having a repeating unit of Formula (2): 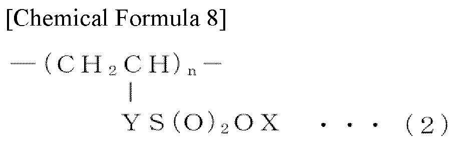
In Formula (2), Y is a bond or an oxygen atom, preferably a bond;
n is an integer of 2 to 5000, preferably 10 to 500, and more preferably 20 to 100; and
X is hydrogen or an alkali metal, preferably hydrogen or Na.

The compound of this embodiment preferably includes polyvinyl sulfonic acid or a salt thereof (e.g., Na salt), or polyvinyl sulfuric acid or a salt thereof (e.g., Na salt); and more preferably, polyvinyl sulfonic acid and a salt thereof having 2 to 5000, more preferably 10 to 500 and still more preferably 20 to 100 of the following repeating structure: 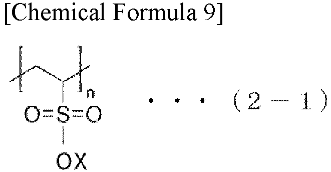 in which X is hydrogen or an alkali metal, preferably hydrogen or Na. Such polyvinyl sulfonic acid or a salt thereof usually has a molecular weight of 200 to 500000, preferably has a molecular weight of 1000 to 50000, and more preferably has a molecular weight of 4000 to 6000.

The term "molecular weight" or "average molecular weight" as used herein means a weight-average molecular weight (Mw) unless otherwise specified. The weight average molecular weight (Mw) as used herein means a value measured by gel permeation chromatography (GPC method) using a liquid chromatograph.

Still another specific embodiment of the organic sulfonic acid, sulfate ester, or salt thereof include a compound having a repeating unit containing the structure of Formula (3): 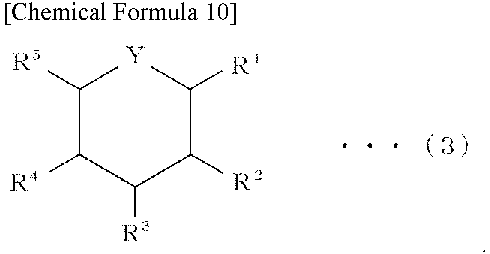
In Formula (3), Y is O or CH₂, preferably O; and
Two of R¹ to R⁵ are O- or a bond, and at least two of the rest are O-S(O)₂OX or S(O)₂OX in which X is hydrogen or an alkali metal (e.g., Na), and those not corresponding to any of the above is OX or C(O)OX in which X is hydrogen or an alkali metal (e.g., Na), preferably C(O)OX in which X is hydrogen or an alkali metal (e.g., Na). Preferably, two of R¹ to R⁵ are O- or a bond, and the rest are O-S(O)₂OX in which X is hydrogen or an alkali metal (e.g., Na).

In addition, the repeating unit containing the above structure includes the following:

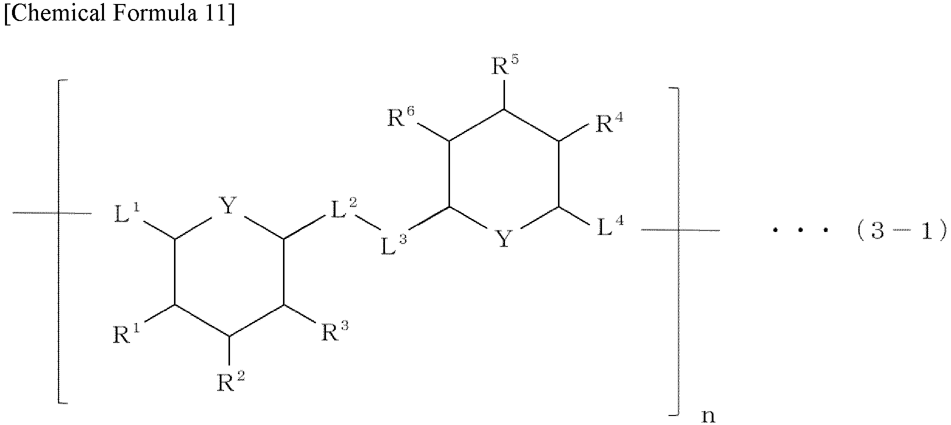

In Formula (3-1), Y is O or CH₂, preferably O;
L¹ and L⁴ are each independently O or a bond; preferably, one of L¹ and L⁴ is O-, and the other is a bond.
L² and L³ are each independently O, CH₂, or a bond; preferably, one of L² and L³ is O, and the other is CH₂ or a bond.
At least two of R¹ to R³ are each independently O-S(O)₂OX or S(O)₂OX in which X is hydrogen or an alkali metal (e.g., Na); and the rest are, if present, OX or C(O)OX in which X is hydrogen or an alkali metal (e.g., Na), preferably C(O)OX in which X is hydrogen or an alkali metal (e.g., Na). R¹ to R³ are, preferably, each independently O-S(O)₂OX or S(O)₂OX in which X is hydrogen or an alkali metal (e.g., Na), and more preferably, each independently O-S(O)₂OX in which X is hydrogen or an alkali metal (e.g., Na).
At least two of R⁴ to R⁶ are each independently O-S(O)₂OX or S(O)₂OX in which X is hydrogen or an alkali metal (e.g., Na); and the rest are, if present, OX or C(O)OX in which X is hydrogen or an alkali metal (e.g., Na), preferably C(O)OX in which X is hydrogen or an alkali metal (e.g., Na). R⁴ to R⁶ are, preferably, each independently O-S(O)₂OX or S(O)₂OX in which X is hydrogen or an alkali metal (e.g., Na), and more preferably, each independently O-S(O)₂OX in which X is hydrogen or an alkali metal (e.g., Na).
n is an integer of 2 to 1000, preferably 3 to 700, more preferably 4 to 200, and still more preferably 5 to 70.

The compound of this embodiment preferably includes dextran sulfate or a salt thereof (e.g., Na), and more preferably dextran sulfate or a salt thereof (e.g., Na) having 2 to 1000, more preferably 3 to 700, and still more preferably 5 to 70 of the following repeating structure: 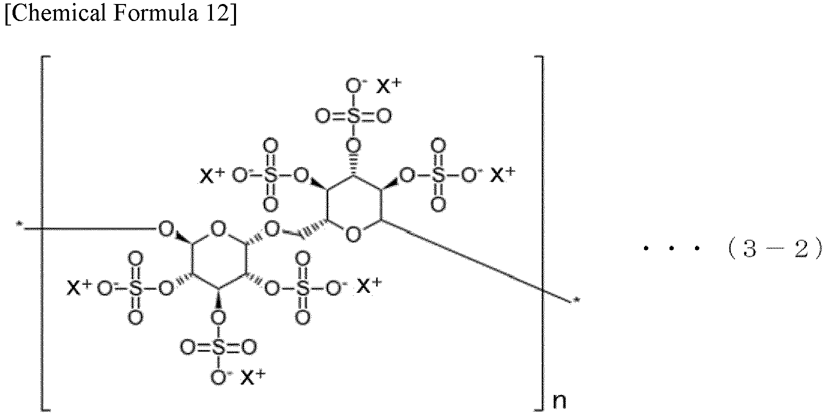 in which X is hydrogen or an alkali metal (e.g., Na). Such dextran sulfate or a salt thereof usually has a molecular weight of 1500 to 1000000, preferably a molecular weight of 2000 to 550000, and more preferably a molecular weight of 4000 to 55000.

In the method of the present invention, the organic sulfonic acid, sulfate ester, or salt thereof described above in a reaction system modifies the antigen property of the coexisting TRACP-5a and inhibits its activity. These TRACP-5a inhibitors can be present in the reaction system in various manners. For example, the above TRACP-5a inhibitors or solutions thereof may be added separately to a reaction solution for detecting TRACP-5b; or may be dissolved in a specimen diluent in advance and added to a measurement sample; or may be added in advance to a reagent containing an enzyme substrate or a reagent containing an anti-TRACP-5b antibody depending on the reaction system, and a reaction (e.g., an enzymatic reaction or an antigen-antibody reaction) may be performed using these reagents.

The organic sulfonic acid, sulfate ester, or salt thereof increases the TRACP-5a inhibitory activity depending on the density of the sulfo group, sulfuric acid group and salt thereof, and the average molecular weight of these compounds; and the lower limit of the concentration of these compounds in the reaction solution required for exhibiting the TRACP-5a inhibitory activity decreases depending on the density and the average molecular weight, as shown in Examples described later. For any of the compounds, as the concentration in the reaction solution is increased from the lower limit value, the inhibitory activity increases depending on the concentration in a certain range; but at or above a certain level of the concentration, the inhibitory activity does not increase depending on the concentration and maintains a certain level; and if the concentration reaches a certain level, the inhibitory activity gradually decreases. However, even in the case that the inhibitory activity reaches a plateau, high inhibitory activity is still maintained in a wide range of concentrations, and practically, the above compounds are often used at a concentration higher than the concentration at which the inhibitory activity does not increase depending on the concentration in the reaction solution.

Therefore, it is preferable to determine the concentration of the organic sulfonic acid, sulfate ester, or salt thereof in the reaction solution in consideration of these points.

For example, in case dextran sulfate or a salt thereof having an average molecular weight of 4000 to 6000 is used, the concentration in the reaction solution is preferably 0.03 × 10⁻³ to 10.0 mass%, more preferably 0.15 × 10⁻³ to 5.0 mass%, and still more preferably 0.3 × 10⁻³ to 1.0 mass%. In case dextran sulfate or a salt thereof having a molecular weight of 40000 to 60000 is used, the concentration in the reaction solution is preferably 0.03 × 10⁻⁴ to 5.0 mass%, more preferably 0.15 × 10⁻⁴ to 1.0 mass%, and still more preferably 0.5 × 10⁻⁴ to 0.5 mass%. In case dextran sulfate or a salt thereof having a molecular weight of 400000 to 600000 is used, the concentration in the reaction solution is preferably 0.01 × 10⁻⁵ to 1.0 mass%, more preferably 0.3 × 10⁻⁴ to 0.1 mass%, and still more preferably 0.5 × 10⁻⁵ to 0.05 mass%.

In case vinyl sulfonic acid or a salt thereof is used, the concentration in the reaction solution is preferably 0.012 to 15.0 mass%, more preferably 0.020 to 5.0 mass%, and still more preferably 0.10 to 3.0 mass%. For example, in case polyvinyl sulfonic acid or a salt thereof having an average molecular weight of 4000 to 6000 is used, the concentration in the reaction solution is preferably 0.08 × 10⁻³ to 5.0 mass%, more preferably 0.3 × 10⁻³ to 1.0 mass%, and still more preferably 0.3 × 10⁻² to 0.3 mass%.

In the measurement method, the above-described organic sulfonic acid, sulfate ester, or salt thereof in the reaction system for detecting TRACP-5b inhibits the TRACP-5a, and the "reaction system" include an enzymatic reaction and an antigen-antibody reaction. The TRACP-5a inhibitor used in the method of the present invention can be used in any of these reaction systems.

As used herein, the "enzymatic reaction" refers to a reaction between TRACP-5b and a substrate thereof. The substrate of TRACP-5b includes p-nitrophenyl phosphate or a salt thereof, 2-chloro-4-nitrophenyl phosphate or a salt thereof, a 1,2-dioxetane derivative, an acridan derivative, and 4-methylumbelliferyl phosphate. As used herein, the "antigen-antibody reaction" refers to a reaction between TRACP-5b and an antibody against the TRACP-5b. The antibody against TRACP-5b include a monoclonal antibody, a polyclonal antibody, and an antigen-binding fragment. The monoclonal antibody or antigen-binding fragment thereof includes an anti-TRACP-5b monoclonal antibody or antigen-binding fragment thereof described in Patent Literatures 6 and 7, and more specifically include an antibody or antigen-binding fragment thereof produced by a hybridoma of accession number IPOD FERM BP-7889, accession number IPOD FERM BP-8249, or accession number IPOD FERM BP-7890.

The methods using an enzymatic reaction and an antigen-antibody reaction include various measurement methods, for example, an enzyme method, enzyme immunoassay (EIA), enzyme-linked immunosorbent assay (ELISA), chemiluminescent enzyme immunoassay (CLEIA method), chemiluminescent immunoassay (CLIA method), and latex agglutination measurement method.

As used herein, the enzyme method refers to a method for measuring the enzymatic activity of TRACP-5b, and for example, TRACP-5b in a specimen is reacted with an enzymatic substrate of TRACP-5b, e.g., P-nitrophenyl phosphate or a salt thereof, to measure the enzymatic activity of TRACP-5b. More specifically, a chromogenic substrate solution (e.g., 2-chloro-4-nitrophenyl phosphate solution) containing tartaric acid or a salt thereof is added to a specimen to start the enzymatic reaction, and the enzymatic activity is determined from the absorbance change between particular time points. In this enzyme method, the above-described organic sulfonic acid, sulfate ester, or salt thereof may be independently added to the reaction solution, the specimen, or the chromogenic substrate solution.

As used herein, the enzyme immunoassay (EIA) refers to an immunoassay for detection using the enzymatic activity of TRACP-5b. For example, TRACP-5b in a specimen is bound to an anti-TRACP-5b monoclonal antibody, and the bound TRACP-5b is reacted with an enzyme substrate of TRACP-5b, e.g., P-nitrophenyl phosphate or a salt thereof, to measure the enzymatic activity of TRACP-5b. More specifically, first, a specimen is brought into contact with an anti-TRACP-5b monoclonal antibody adsorbed on a solid support, and TRACP-5b in the specimen is bound to the immobilized antibody. Next, the solid support is washed with a wash solution to remove specimen-derived components which have not been adsorbed onto the antibody; and then an enzymatic substrate of TRACP-5b, e.g., p-nitrophenyl phosphate or a salt thereof, is added to the reaction system to react the TRACP-5b bound to the antibody with the substrate. After stopping the enzymatic reaction with a reaction terminating solution, the absorbance of a phenol compound produced by the reaction, e.g., p-nitrophenol, is measured at a predetermined wavelength, e.g., a wavelength of 390 nm to 450 nm, preferably 400 to 430 nm. The magnitude of the absorbance reflects the enzymatic activity of TRACP-5b, and therefore, TRACP-5b in the specimen can be determined in accordance with the absorbance value. In this enzyme immunoassay (EIA), the above-described organic sulfonic acid, sulfate ester, or salt thereof may be independently added to the support, the specimen or a specimen diluent, a reagent containing the anti-TRACP-5b monoclonal antibody, or a chromogenic substrate solution.

As used herein, the enzyme-linked immunosorbent assay (ELISA) refers to a method of measuring TRACP-5b through an enzymatic reaction using two different antibodies against TRACP-5b, one of which is an antibody labeled with a substance enabling detection through the enzymatic reaction. For example, a specimen is brought into contact with a primary anti-TRACP-5b monoclonal antibody adsorbed on a solid support to bind TRACP-5b in the specimen to the primary antibody, then a secondary anti-TRACP-5b monoclonal antibody that is capable of binding to the TRACP-5b at an epitope different from that of the primary antibody and that is labeled with a substance enabling detection through an enzymatic reaction is brought into contact with the immobilized TRACP-5b to bind the secondary antibody to the TRACP-5b, and the RACP-5b is measured through the enzymatic reaction utilizing the label of the secondary antibody.

More specifically, first, a primary anti-TRACP-5b monoclonal antibody is adsorbed onto a solid support, and a specimen is brought into contact with the immobilized primary antibody to bind TRACP-5b in the specimen to the primary antibody. Next, the solid support is washed with a wash solution to remove specimen-derived components which have not been adsorbed onto the antibody; and then a secondary anti-TRACP-5b monoclonal antibody or a polyclonal antibody that is labeled with a substance enabling detection through an enzymatic reaction, e.g., an enzyme or biotin, and that binds to TRACP-5b at an epitope different from that of the primary antibody is brought into contact with the immobilized TRACP-5b to bind the secondary antibody to the TRACP-5b. Next, the label of the secondary antibody is allowed to generate a color substance by, for example, reacting biotin with peroxidase-labeled streptavidin, followed by a peroxidase enzymatic reaction; or reacting a labeling enzyme with its substrate; and the absorbance of a color substance is measured, whereby the TRACP-5b can be measured. The enzyme used as the substance enabling detection through an enzymatic reaction includes peroxidase and alkaline phosphatase, and the substance enabling detection through the enzymatic reaction other than the enzyme includes avidin and substrates of various enzymes. When avidin is used, ABC-based color development may be utilized.

In this enzyme-linked immunosorbent assay (ELISA), the above-described organic sulfonic acid, sulfate ester, or salt thereof may be independently added to the support, the specimen or a specimen diluent, a reagent containing the primary antibody and/or the secondary antibody, or a chromogenic substrate solution.

As used herein, the chemiluminescent enzyme immunoassay (CLEIA method) refers to an immunoassay for detecting the enzymatic activity of TRACP-5b through chemiluminescence. For example, a specimen is brought into contact with an anti-TRACP-5b monoclonal antibody adsorbed on a solid support, such as beads, to bind TRACP-5b in the specimen to the antibody, a chemiluminescent substrate of TRACP-5b is brought into contact with the immobilized TRACP-5b, and generated chemiluminescence is measured, thereby measuring the TRACP-5b activity. More specifically, first, a specimen is brought into contact with an anti-TRACP-5b monoclonal antibody adsorbed on a solid support such as magnetic particles to bind TRACP-5b in the specimen to the immobilized antibody. Next, the solid support is immobilized, for example, by magnetic force of the magnetic particles and washed with a wash solution to remove specimen-derived components which have not been adsorbed onto the antibody; and then a chemiluminescent substrate of TRACP-5b, for example, 2-chloro-5-{4-methoxyspiro[1,2-dioxetane-3,2'-(5'-chloro)tricyclo[3.3.1.13,7]decane]-4-yl} phenyl phosphate disodium salt (CDP-Star, registered trademark) is added to the reaction system to react the TRACP-5b bound to the antibody with the substrate and measure the luminescence intensity. The luminescence intensity reflects the enzymatic activity of TRACP-5b, and therefore, TRACP-5b in the specimen can be measured in accordance with the luminescence value.

In this chemiluminescent enzyme immunoassay (CLEIA method), the above-described organic sulfonic acid, sulfate ester, or salt thereof may be independently added to the support, the specimen or a specimen diluent, a reagent containing the anti-TRACP-5b monoclonal antibody, or a chromogenic substrate solution.

The chemiluminescent enzyme immunoassay (CLEIA method) can also involve the following procedure: a specimen is brought into contact with a primary anti-TRACP-5b monoclonal antibody immobilized on a solid support to bind TRACP-5b in the specimen to the primary antibody, then a secondary anti-TRACP-5b monoclonal antibody that is capable of binding to TRACP-5b at an epitope different from that of the primary antibody and that is labeled with a substance enabling detection with a chemiluminescent substrate or through chemiluminescence is brought into contact with the immobilized TRACP-5b to bind the TRACP-5b to the secondary antibody, thereby generating chemiluminescence from the label of the secondary antibody or by utilizing the label, and the luminescence intensity is measured to measure the TRACP-5b. More specifically, first, a primary anti-TRACP-5b monoclonal antibody is adsorbed onto a solid support, e.g., magnetic particles, and the specimen is brought into contact with the immobilized primary antibody to bind TRACP-5b in the specimen to the primary antibody. Next, the solid support is immobilized, for example, by magnetic force of the magnetic particles, and washed with a wash solution to remove specimen-derived components which have not been adsorbed onto the antibody; and then the secondary anti-TRACP-5b monoclonal antibody or a polyclonal antibody that is labeled with a substance capable of generating chemiluminescence using an enzyme or an enzymatic reaction and that is capable of binding to TRACP-5b at an epitope different from that of the primary antibody is brought into contact with the immobilized TRACP-5b to bind the secondary antibody to the TRACP-5b; and then chemiluminescence is generated from the label of the secondary antibody or by utilizing it; and the luminescence intensity is measured, thereby measuring the TRACP-5b.

In this chemiluminescent enzyme immunoassay (CLEIA method), the above-described organic sulfonic acid, sulfate ester, or salt thereof may be independently added to the support, the specimen, or a reagent containing the primary antibody and/or the secondary antibody.

As used herein, the latex agglutination method refers to a method for measuring TRACP-5b by measuring turbidity generated by agglutination of latex particles as absorbance, wherein, for example, a specimen is brought into contact with an anti-TRACP-5b polyclonal antibody adsorbed on the latex particles to cause agglutination of the latex particles via TRACP-5b, and the turbidity (absorbance) generated by the agglutination is measured to measure the TRACP-5b. In this latex agglutination method, the above-described organic sulfonic acid, sulfate ester, or salt thereof may be independently added to the reaction solution, the specimen, or a reagent containing the latex particles.

A specimen to be measured for TRACP-5b may be any living body-derived specimen, but is usually a mammal-derived specimen, and is often a human-derived specimen. In addition, the specimen may be derived from any tissue or body fluid, but blood, serum, and plasma are often used.

### 2. Kit for measuring TRACP-5b

Another embodiment of the present invention relates to a kit for measuring TRACP-5b, and the kit is characterized by comprising the aforementioned organic sulfonic acid, sulfate ester, and/or salt thereof.

The organic sulfonic acid, sulfate ester, or salt thereof can be comprised in the kit 1) as an independent solution, 2) by being contained in a specimen diluent, 3) by being contained in a substrate-containing reagent, or 4) by being contained in a reagent containing an anti-TRACP-5b antibody, depending on the kit components and measurement principle.

The concentration of the organic sulfonic acid, sulfate ester, or salt thereof in each solution or reagent is preferably adjusted depending on the density of the sulfo group (-S(O)₂OH), sulfuric acid group (-OS(O)₂OH) and/or salt thereof of the organic sulfonic acid, sulfate ester or salt thereof, the molecular weight of these compounds, and the measurement protocol (particularly, dilution factor (the amount of the reaction solution (vol)/the amount of the solution or reagent in which the organic sulfonic acid, sulfate ester, or salt thereof is dissolved or added (vol)) so as to be the concentration in the reaction solution described above.

For example, in case dextran sulfate or a salt thereof having an average molecular weight of 4000 to 6000 is used, the concentration (C) in the solution or reagent is preferably 0.03 × 10⁻³ to 10.0 mass%, more preferably 0.15 × 10⁻³ to 5.0 mass%, and still more preferably 0.3 × 10⁻³ to 1.0 mass%. In case dextran sulfate or a salt thereof having a molecular weight of 40000 to 60000 is used, the concentration in the solution or reagent is preferably 0.03 × 10⁻⁴ to 5.0 mass%, more preferably 0.15 × 10⁻⁴ to 1.0 mass%, and still more preferably 0.5 × 10⁻⁴ to 0.5 mass%. In case dextran sulfate or a salt thereof having a molecular weight of 400000 to 600000 is used, the concentration in the solution or reagent is preferably 0.01 × 10⁻⁵ to 1.0 mass%, more preferably 0.3 × 10⁻⁴ to 0.1 mass%, and still more preferably 0.5 × 10⁻⁵ to 0.05 mass%.

In case vinyl sulfonic acid or a salt thereof is used, the concentration (C) in the solution or reagent is preferably 0.012 to 15.0 mass%, more preferably 0.020 to 5.0 mass%, and still more preferably 0.10 to 3.0 mass%. For example, in case polyvinyl sulfonic acid or a salt thereof having an average molecular weight of 4000 to 6000 is used, the concentration (C) in the solution or reagent is preferably 0.08 × 10⁻³ to 5.0 mass%, more preferably 0.3 × 10⁻³ to 1.0 mass%, and still more preferably 0.3 × 10⁻² to 0.3 mass%.

As mentioned above, TRACP-5b can be measured by various methods, and the kit for measuring TRACP-5b has different reagents depending on the measurement method.

For example, a kit for measuring TRACP-5b by the enzyme method comprises a substrate of TRACP-5b; the aforementioned organic sulfonic acid, sulfate ester, or salt thereof; and optionally a specimen diluent. In this kit, the organic sulfonic acid, sulfate ester, or salt thereof can be contained in an independent solution, or a reagent containing the substrate of TRACP-5b, or the specimen diluent. As the independent solution or the specimen diluent, for example, a buffer such as Tris can be used to dissolve the organic sulfonic acid, sulfate ester, or salt thereof.

A kit for measuring TRACP-5b by the enzyme immunoassay (EIA) comprises a solid support such as multi-well plate; an anti-TRACP-5b monoclonal antibody adsorbed on the solid support; a substrate of TRACP-5b; and the aforementioned organic sulfonic acid, sulfate ester, or salt thereof; optionally a specimen diluent; and optionally a wash solution.

The anti-TRACP-5b monoclonal antibody may be comprised in the kit as an antibody solution separately from the solid support, and in this case, the antibody is adsorbed onto the solid support when TRACP-5b is measured. The wash solution can be used, after TRACP-5b in a specimen is bound to the antibody, to remove components in the specimen that have not been adsorbed onto the antibody. As the wash solution, for example, a Tris buffer containing a surfactant can be used.

The specimen diluent is used for adjusting the concentration of the specimen, and for example, a buffer such as Tris can be used. In addition, the buffer may be optionally supplemented with a chelating agent such as EDTA-2Na or an inorganic salt such as NaCl.

The material of the solid support includes polystyrene, polypropylene, polycarbonate, polyethylene, nylon, and methacrylate. The shape of the solid support includes a plate and (magnetic) beads (particles).

In this kit, the organic sulfonic acid, sulfate ester, or salt thereof can be contained in an independent solution, or a reagent containing the substrate of TRACP-5b, or a reagent containing the anti-TRACP-5b monoclonal antibody, or the specimen diluent. As the independent solution or the specimen diluent, for example, a buffer such as Tris can be used to dissolve the organic sulfonic acid, sulfate ester, or salt thereof.

The kit for measuring TRACP-5b by the enzyme-linked immunosorbent assay (ELISA) comprises a solid support; a primary anti-TRACP-5b monoclonal antibody immobilized on the solid support; a secondary anti-TRACP-5b monoclonal antibody that is capable of binding to TRACP-5b at an epitope different from that of the primary anti-TRACP-5b monoclonal antibody and that is labeled with a substance enabling detection through an enzymatic reaction; a reagent for detecting the substance of labeling the secondary anti-TRACP-5b monoclonal antibody through the enzymatic reaction; the aforementioned organic sulfonic acid, sulfate ester, or salt thereof; optionally a specimen diluent; and optionally a wash solution.

The primary anti-TRACP-5b monoclonal antibody may be comprised in the kit as an antibody solution separately from the solid support, and in this case, the primary antibody is adsorbed onto the solid support when TRACP-5b is measured. The wash solution is used, after TRACP-5b in a specimen is bound to the primary antibody or the secondary antibody, to remove components that have not been adsorbed to the antibodies. As the wash solution, for example, a Tris buffer containing a surfactant can be used.

The specimen diluent is used for adjusting the concentration of the specimen, and for example, a buffer such as Tris can be used. In addition, the buffer may be optionally supplemented with a chelating agent such as EDTA-2Na and an inorganic salt such as NaCl.

In this kit, the organic sulfonic acid, sulfate ester, or salt thereof can be contained in an independent solution, or a reagent for enabling detection of the label of the secondary anti-TRACP-5b monoclonal antibody through an enzymatic reaction, a reagent containing the primary antibody or the secondary antibody, or the specimen diluent. As the independent solution or the specimen diluent, for example, a buffer such as Tris can be used to dissolve the organic sulfonic acid, sulfate ester, or salt thereof.

The substance enabling detection through an enzymatic reaction includes enzymes other than ACP, substrates of enzymes other than ACP, or biotin.

For example, in case the labeling substance is biotin, a reagent for detecting the labeling substance through an enzymatic reaction may contain peroxidase-labeled streptavidin, a substrate of peroxidase enzyme, e.g., a tetramethylbenzidine, and hydrogen peroxide. In case the labeling substance is an enzyme, the reagent contains a substrate of the enzyme. For example, when the labeling substance is alkaline phosphatase, the reagent contains p-nitrophenyl phosphate or the like as a substrate of the enzyme.

The material of the solid support includes polystyrene, polypropylene, polycarbonate, polyethylene, nylon, and methacrylate. The shape of the solid support includes a plate and (magnetic) beads (particles).

A kit for measuring TRACP-5b by the chemiluminescent enzyme immunoassay (CLEIA method) comprises, for example, a solid support; an anti-TRACP-5b monoclonal antibody adsorbed on the solid support; a chemiluminescent substrate of TRACP-5b; the aforementioned organic sulfonic acid, sulfate ester, or salt thereof; optionally a specimen diluent; and optionally a wash solution.

The anti-TRACP-5b monoclonal antibody may be comprised in the kit as an antibody solution separately from the solid support, and in this case, the antibody is adsorbed onto the solid support when TRACP-5b is measured.

The chemiluminescent substrate includes:
(1) 1,2-dioxetane derivatives such as 3-(4-methoxyspiro{1,2-dioxetane-3,2-(5'-chloro)tricyclo[3.3.1.13,7]decane }-4-yl)phenyl phosphate disodium salt, 4-chloro-3-(methoxyspiro{1,2-dioxetane-3,2'-(5'-chloro)tricyclo[3.3.1.13,7]decane}-4-yl)phenylphosphate disodium salt, 3-(4-methoxyspiro{1,2-dioxetane-3,2'-tricyclo[3.3.1.1(3,7)]decane}-4-yl)phenyl phosphate disodium salt, and 4-methoxy-4-(3-phenyl phosphate)spiro[1,2-dioxetane-3,2'-adamantane]disodium salt; or
(2) acridan derivatives such as [10-methyl-9(10H)-acridinylidene]phenoxymethyl phosphate disodium salt.

The specimen diluent is used for adjusting the concentration of a specimen, and for example, the following can be used: a buffer selected from the group consisting of a 2-(N-morpholino)ethanesulfonic acid (MES) buffer, 3-(N-morpholino)propanesulfonic acid (MOPSO) buffer, 3-morpholinopropanesulfonic acid (MOPS) buffer, N,N-bis(2-hydroxyethyl)-2-aminoethanesulfonic acid (BES) buffer, N-tris(hydroxymethyl)methyl-2-aminoethanesulfonic acid (TES) buffer, 2-[4-(2-hydroxyethyl)-1-piperazinyl]ethanesulfonic acid (HEPES) buffer, N-(2-acetamide)iminodiacetic acid (ADA) buffer, N-(2-acetamide)-2-aminoethanesulfonic acid (ACES) buffer, bis(2-hydroxyethyl)iminotris(hydroxymethyl)methane (Bis-Tris) buffer, tris(hydroxymethyl)aminomethane (Tris) buffer, and phosphate buffer. In addition, the buffer may be optionally supplemented with cobalt ions or manganese ions as metal ions.

The wash solution is used, after TRACP-5b in a specimen is bound to the antibody, to remove components in the specimen that have not been adsorbed onto the antibody. As the wash solution, for example, the above-described buffer containing a surfactant can be used.

In this kit, the organic sulfonic acid, sulfate ester, or salt thereof can be contained in an independent solution, or a reagent containing a chemiluminescent substrate of TRACP-5b, or a reagent containing the anti-TRACP-5b monoclonal antibody, or the specimen diluent. As the independent solution or the specimen diluent, for example, the above-described buffer can be used to dissolve the organic sulfonic acid, sulfate ester, or salt thereof.

A kit for measuring TRACP-5b by the chemiluminescent immunoassay (CLIA method) also comprises a solid support; a primary anti-TRACP-5b monoclonal antibody adsorbed on the solid support; a secondary anti-TRACP-5b monoclonal antibody that is capable of binding to TRACP-5b at an epitope different from that of the primary anti-TRACP-5b monoclonal antibody and that is labeled with a substance enabling detection through chemiluminescence; and a reagent enabling detection of the label through chemiluminescence; the organic sulfonic acid, sulfate ester, or salt thereof; optionally a specimen diluent; and optionally a wash solution.

The primary anti-TRACP-5b monoclonal antibody may be comprised in the kit as an antibody solution separately from the solid support, and in this case, the primary antibody is adsorbed onto the solid support when TRACP-5b is measured. The wash solution is used, after TRACP-5b in a specimen is bound to the primary antibody or the secondary antibody, to remove components that have not been adsorbed to the antibodies. The wash solution and the specimen diluent are the same as described above.

For example, in case the label is biotin, the reagent enabling detection of the label through chemiluminescence is a reagent containing peroxidase-labeled streptavidin, a peroxidase chemiluminescent substrate, and hydrogen peroxide; and in case the label is an enzyme, e.g., alkaline phosphatase, the reagent contains a substrate of the enzyme, such as p-nitrophenyl phosphate. The chemiluminescent substrate is the same as described above.

The material of the solid support includes polystyrene, polypropylene, polycarbonate, polyethylene, nylon, and methacrylate. The shape of the solid support includes a plate and (magnetic) beads (particles).

In this kit, the organic sulfonic acid, sulfate ester, or salt thereof can be contained in an independent solution, or the reagent enabling detection of the label through chemiluminescence, a reagent containing the primary antibody or the secondary antibody, or the specimen diluent. As the independent solution or the specimen diluent, for example, the above-described buffer can be used to dissolve the organic sulfonic acid, sulfate ester, or salt thereof.

A kit for measuring TRACP-5b by the latex agglutination method comprises latex particles; an anti-TRACP-5b polyclonal antibody or an anti-TRACP-5b monoclonal antibody adsorbed on the latex particles; the aforementioned organic sulfonic acid, sulfate ester, or salt thereof; and optionally a specimen diluent. In this kit, the organic sulfonic acid, sulfate ester, or salt thereof can be contained in an independent solution, or a reagent containing the latex particles, or the specimen diluent. As the independent solution or the specimen diluent, for example, a buffer can be used to dissolve the organic sulfonic acid, sulfate ester, or salt thereof. The buffer can include a 2-(N-morpholino)ethanesulfonic acid (MES) buffer, 3-(N-morpholino)propanesulfonic acid (MOPSO) buffer, 3-morpholinopropanesulfonic acid (MOPS) buffer, N,N-bis(2-hydroxyethyl)-2-aminoethanesulfonic acid (BES) buffer, N-tris(hydroxymethyl)methyl-2-aminoethanesulfonic acid (TES) buffer, 2-[4-(2-hydroxyethyl)-1-piperazinyl]ethanesulfonic acid (HEPES) buffer, N-(2-acetamide)iminodiacetic acid (ADA) buffer, N-(2-acetamide)-2-aminoethanesulfonic acid (ACES) buffer, bis(2-hydroxyethyl)iminotris(hydroxymethyl)methane (Bis-Tris) buffer, tris(hydroxymethyl)aminomethane (Tris) buffer, and phosphate buffer.

### Examples

Hereinafter, the present invention will be described with reference to Examples, but the present invention is not limited thereto.

The meanings of the abbreviations used in Examples are as follows.

| | |
|---|---|
| DSS Na 5k | Dextran sulfate sodium having an average molecular weight of 5000 |
| DSS Na 50k | Dextran sulfate sodium having an average molecular weight of 50000 |
| DSS Na 500k | Dextran sulfate sodium having an average molecular weight of 500000 |
| PVSA Na 5k | Sodium polyvinyl sulfonate having an average molecular weight of 5000 |
| VSA Na | Sodium vinylsulfonate |
| β-CyD-S Na | β-cyclodextrin sodium sulfate |
| PAA 5k | Polyacrylic acid having an average molecular weight of 5000 |
| Poly-Glu Na | Sodium polyglutamate |
| Chon S Na | Sodium chondroitin sulfate |
| PEG 6k | Polyethylene glycol having an average molecular weight of 6000 |

### Test 1. Evaluation of TRACP-5a inhibitor in an enzyme method

### 1-1. Evaluation compounds

The following compounds were evaluated for the inhibitory effect on TRACP-5a in an enzyme method.

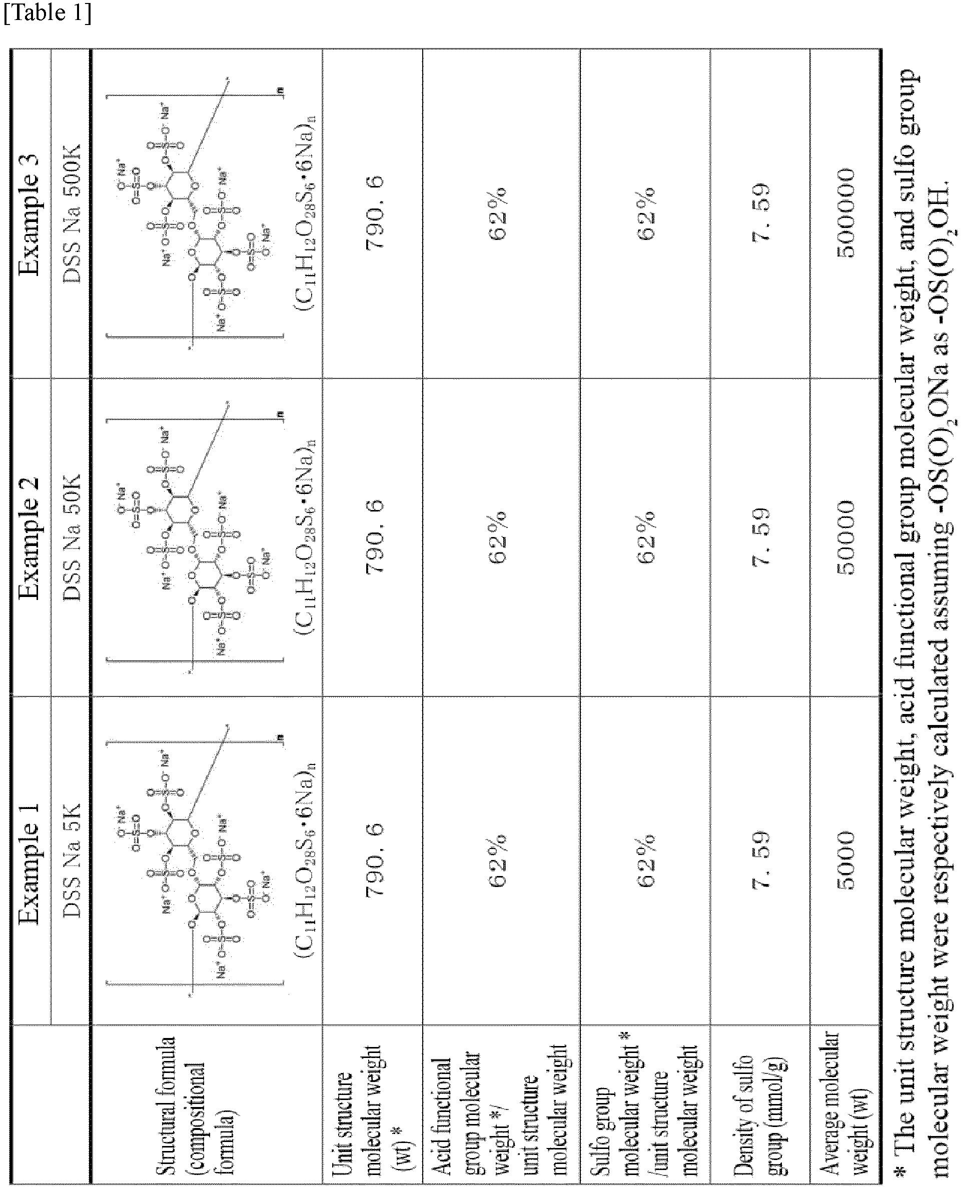

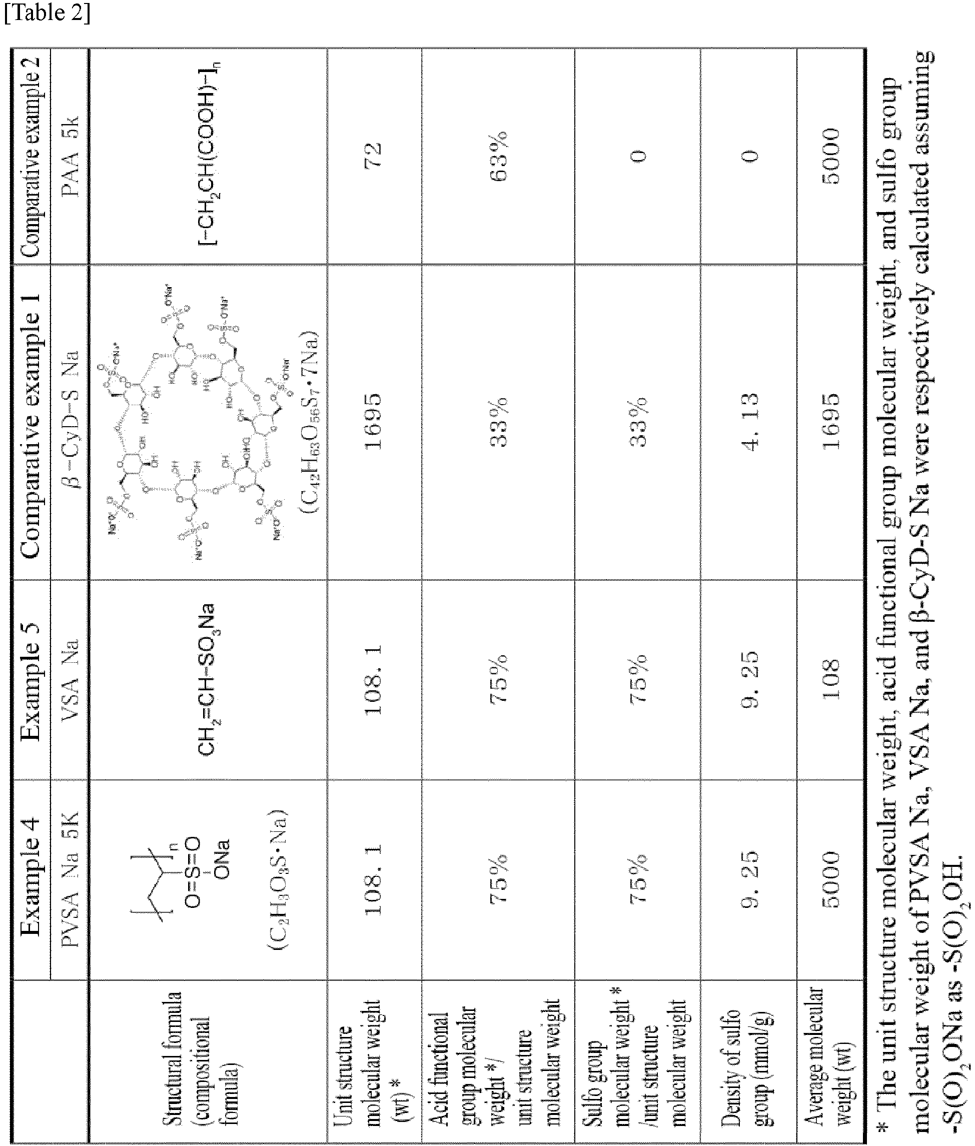

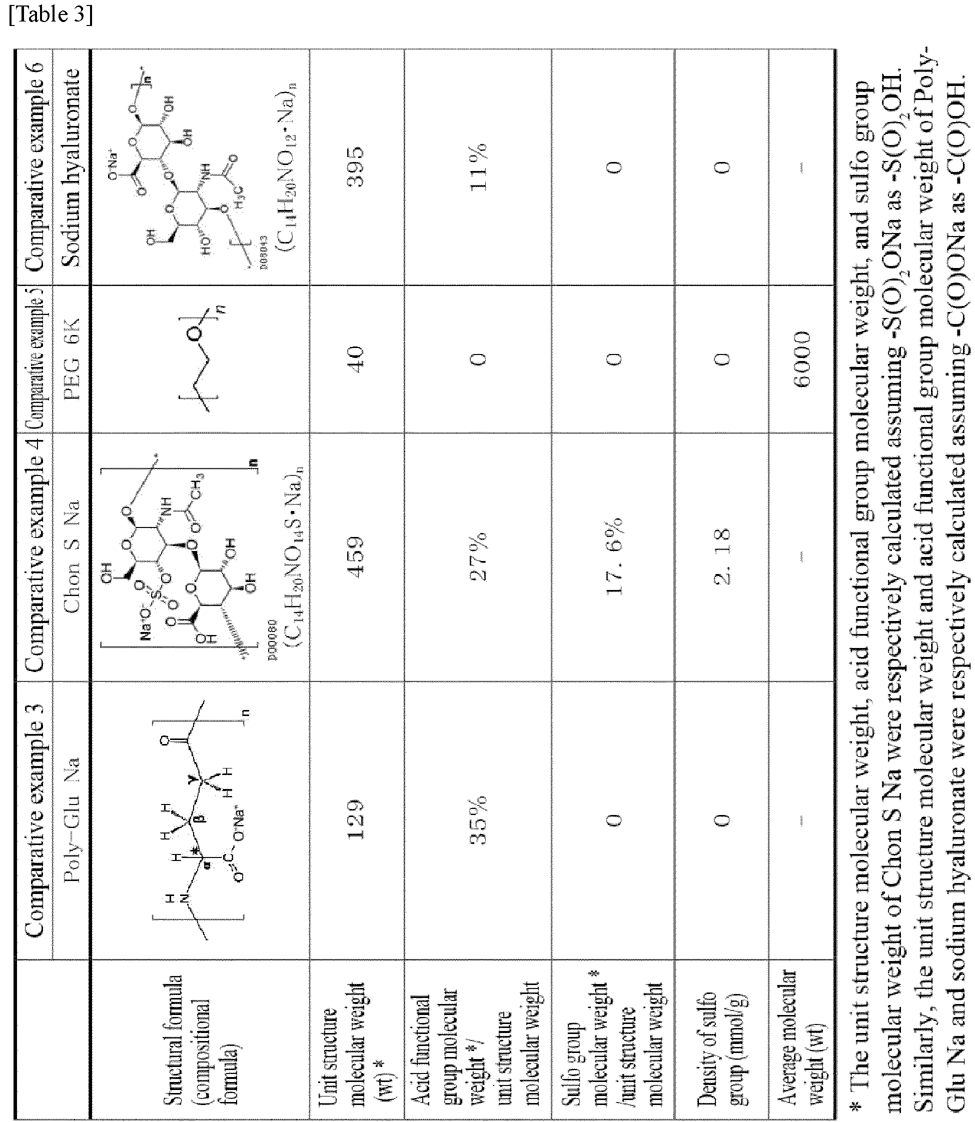

### 1-2. Measurement sample

TRACP-5b manufactured by Nittobo Medical Co., Ltd. and TRACP-5a manufactured by R&D Systems, Inc. were diluted with a 22 mM MES buffer (pH 5.0) to prepare a 7 U/L TRACP-5b solution and a 7 U/L TRACP-5a solution, and the solutions were used as measurement samples. In the measurement with sodium hyaluronate, a 7 U/L TRACP-5b solution and an 8 U/L TRACP-5a solution prepared in the same manner were used.

### 1-3. Measurement reagent (chromogenic substrate solution)

A chromogenic substrate solution having the following composition was used.
5 mM 2-chloro-4-nitrophenyl phosphate
109 mM MES
40 mM sodium tartrate
0.1 mass% evaluation compound
pH 6.6

### 1-4. Measurement method

For the measurement, Hitachi automatic analyzer model 7180 (Hitachi High-Tech Corporation) was used. To 16 µL of the 7 U/L TRACP-5b solution, or the 7 U/L TRACP-5a solution or the 8 U/L TRACP-5a solution, 200 µL of the chromogenic substrate solution containing an evaluation compound was added to start an enzymatic reaction, and the absorbance (primary wavelength: 405 nm, secondary wavelength: 480 nm) was measured for 516.0 seconds starting at 340.5 seconds after the beginning of the enzymatic reaction. The amount of absorbance change per minute (the amount of absorbance change per unit time during the enzymatic reaction) was determined from the absorbance change between both the time points.

On the other hand, serial dilutions of a TRACP-5b reference solution with known activity in a 22 mM MES buffer solution (pH 5.0) as well as a 22 mM MES buffer solution (pH 5.0) as a blank were prepared; the serial dilutions of the TRACP-5b reference solution were measured using the chromogenic substrate solution containing each evaluation compound, thereby producing a standard straight line for the TRACP-5b. In addition, the serial dilutions of the TRACP-5b reference solution were measured using the chromogenic substrate solution without any evaluation compound in the same manner, thereby producing a standard straight line for the TRACP-5b. The chromogenic substrate solution containing each evaluation compound and the chromogenic substrate solution without any evaluation compound were used to measure the TRACP-5b solution and the TRACP-5a solution, and these standard straight lines were used to calculate activity values from the obtained amounts of absorbance change.

The measurement using sodium hyaluronate was performed in a separate batch from that with other evaluation compounds.

### 1-5. Measurement results

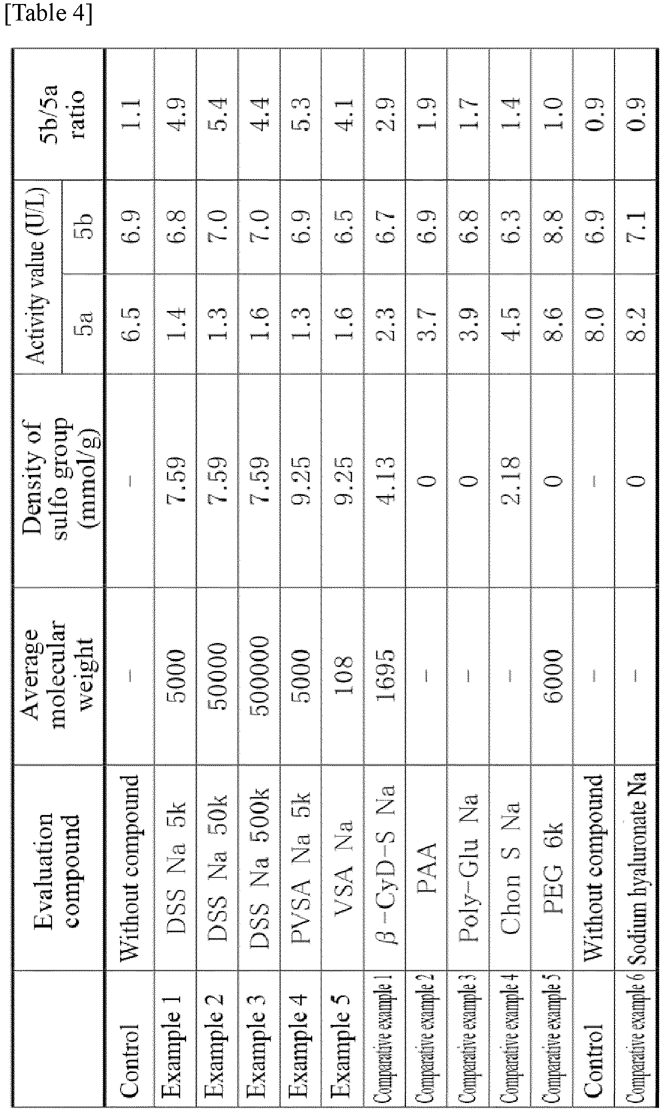

### Test 2. Study of concentration of TRACP-5a inhibitor

For dextran sulfate sodium (5K), dextran sulfate sodium (50K), sodium polyvinyl sulfonate (5K), and sodium vinylsulfonate, the influence of the concentration on the inhibitory activity was studied.

### 2-1. Concentration of each evaluation compound

The concentration of each evaluation compound in the measurement reagent (chromogenic substrate solution) was adjusted as follows.

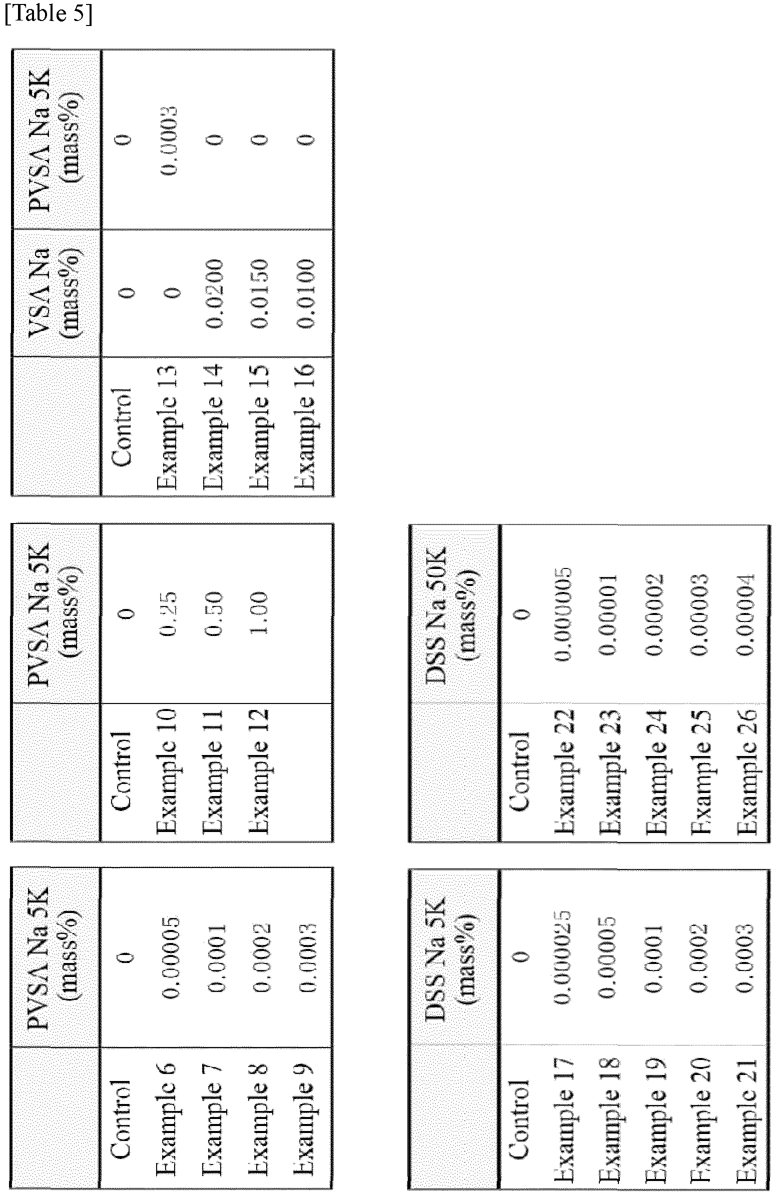

### 2-2. Measurement sample

A 7 U/L TRACP-5b solution and a 7 U/L TRACP-5a solution were used in the test using a reagent containing dextran sulfate sodium (5K) and dextran sulfate sodium (50K); a 7 U/L TRACP-5b solution and a 7 U/L or 8 U/L TRACP-5a solution were used in the test using a reagent containing sodium polyvinyl sulfonate (5K); and a 7 U/L TRACP-5b solution and an 8 U/L TRACP-5a solution were used in the test using a reagent containing sodium vinylsulfonate.

### 2-3. Measurement reagent (chromogenic substrate solution) and measurement method

The chromogenic substrate solution having the same composition as in Test 1 was used, except that the concentration of each evaluation compound was as described above. The measurement method was carried out in the same procedure as in Test 1.

### 2-4. Measurement results

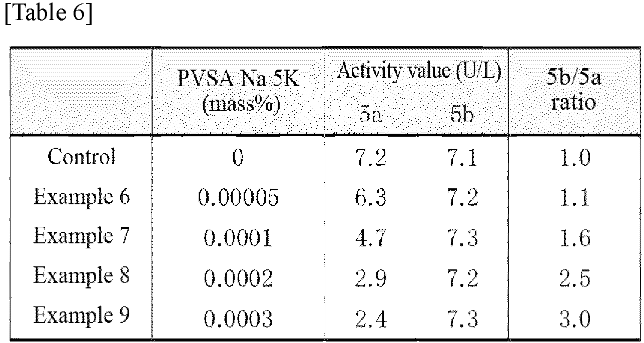

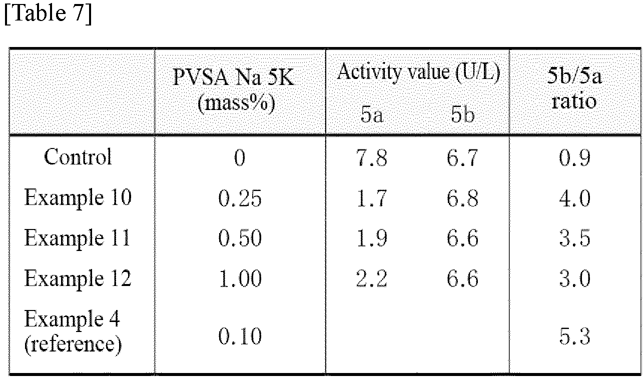

The data of Example 4 is from another batch, but is included herein from Test 1 as a reference.

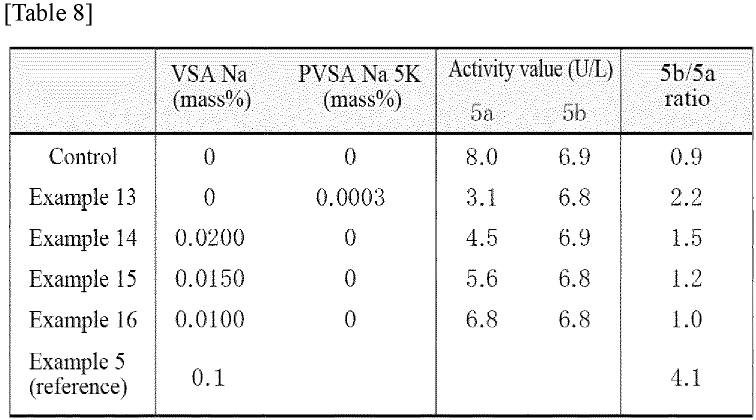

The data of Example 5 is from another batch, but is included herein from Test 1 as a reference.

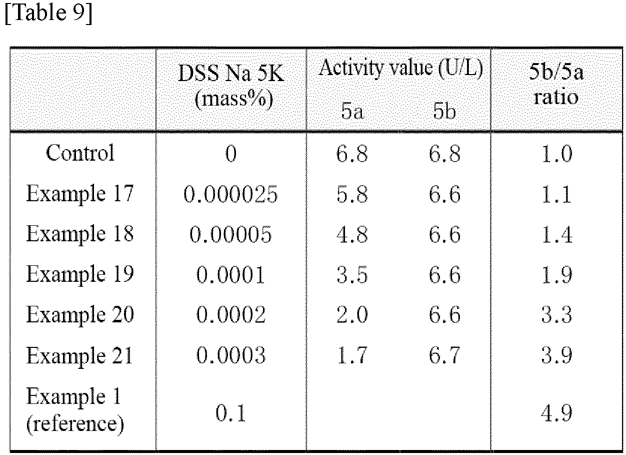

The data of Example 1 is from another batch, but is included herein from Test 1 as a reference.

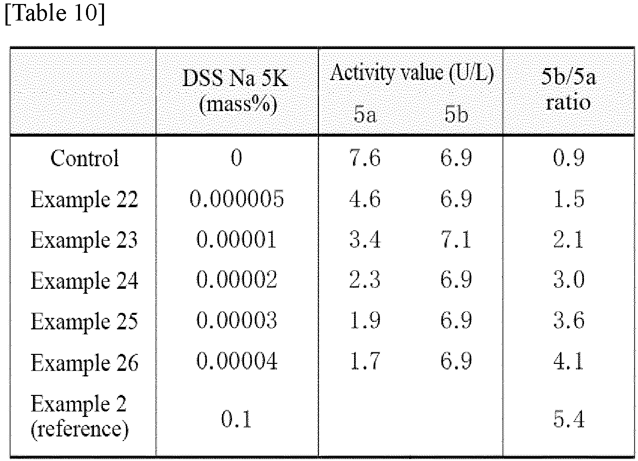

The data of Example 2 is from another batch, but is included herein from Test 1 as a reference.

### 3. Evaluation of TRACP-5a inhibitor in EIA method

### 3-1. Evaluation compounds

The following compounds were evaluated for the inhibitory effect on TRACP-5a in an EIA method.

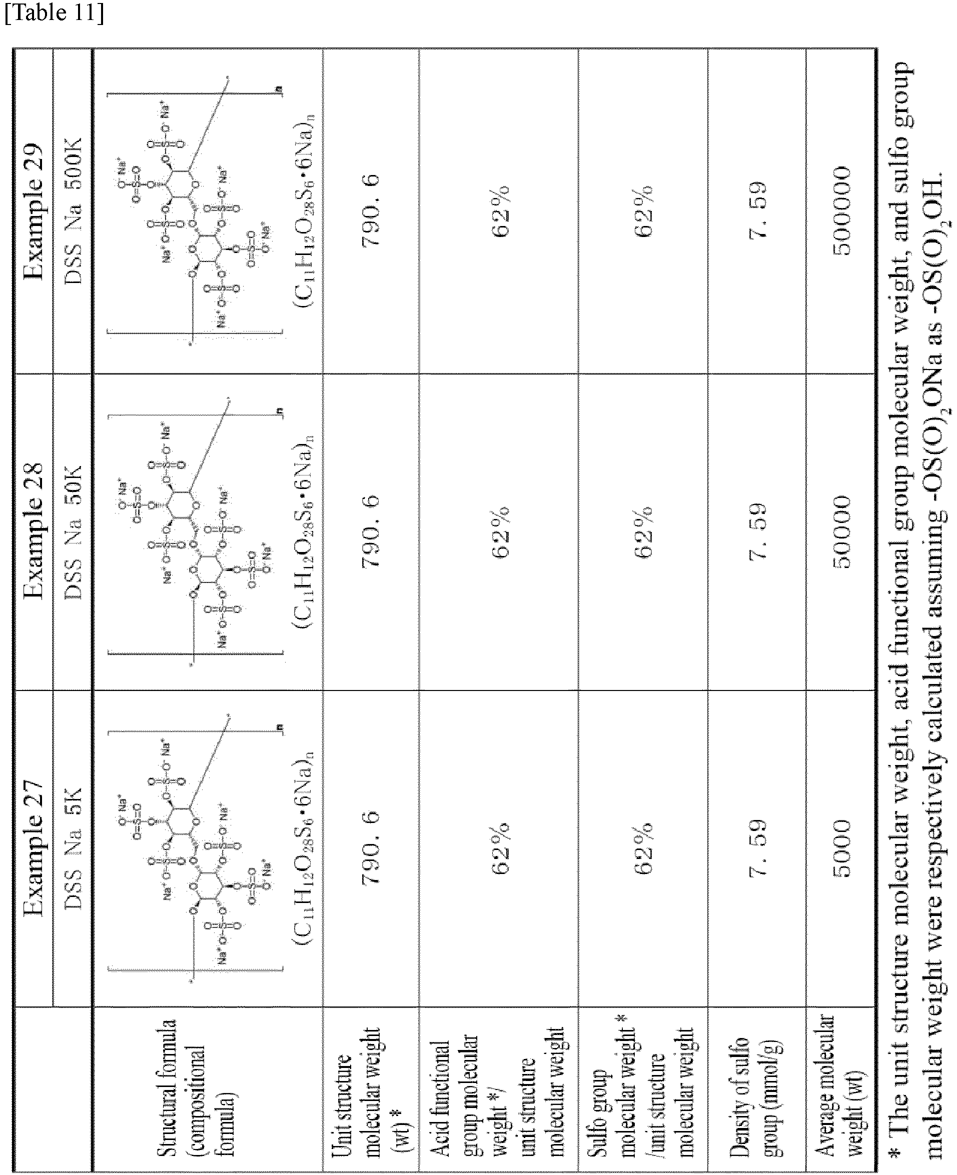

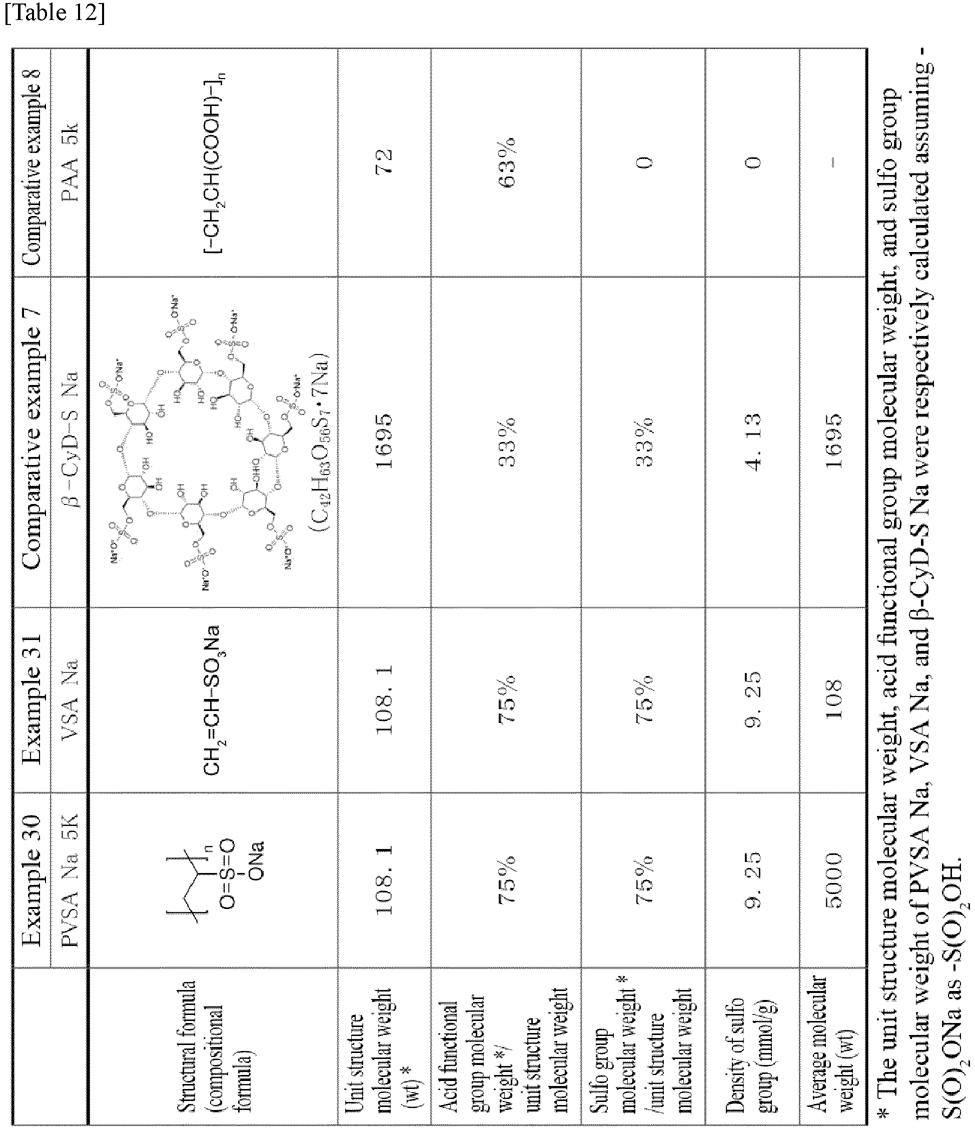

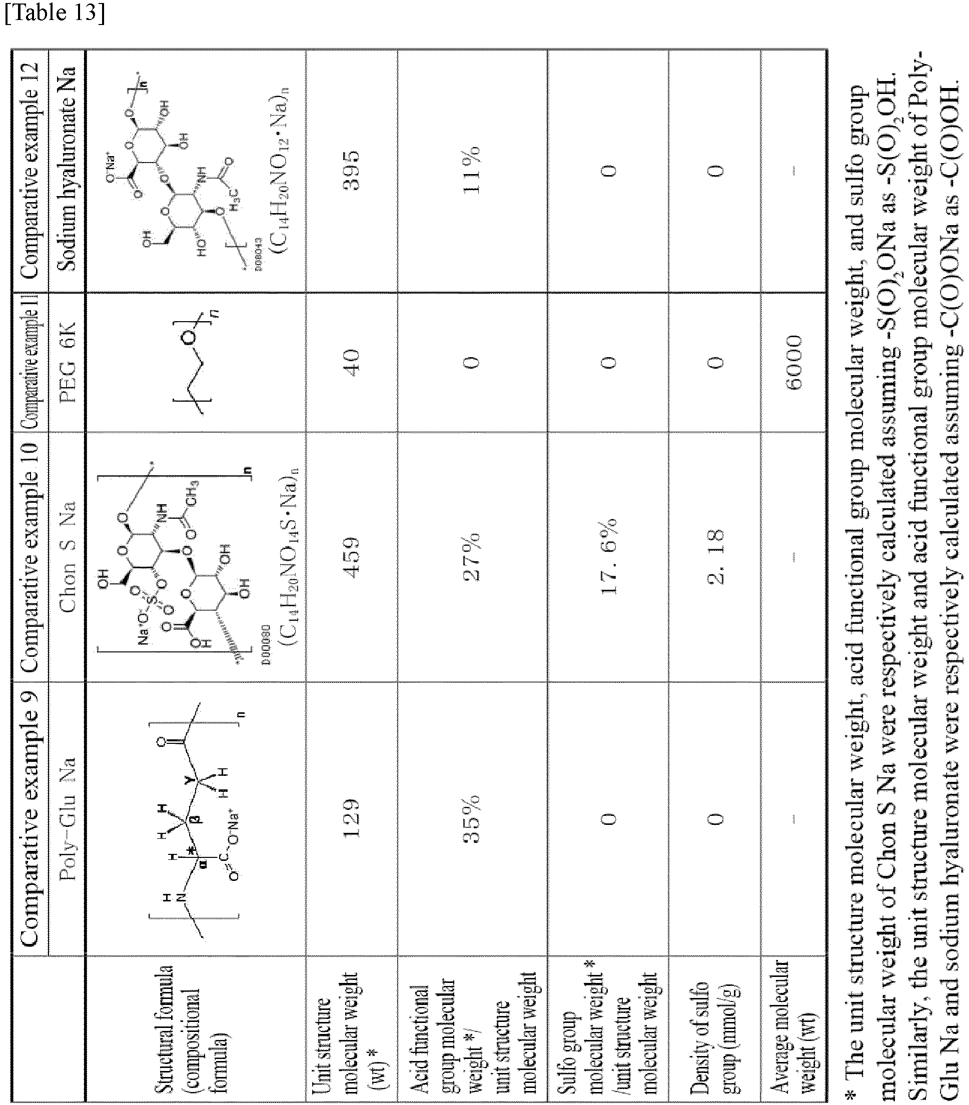

### 3-2. Preparation of antibody-immobilized plate

A TRACP-5b monoclonal antibody (Accession Number IPOD FERM BP-7890) was diluted with PBS (8 mM disodium hydrogenphosphate, 1.5 mM sodium dihydrogenphosphate, 2.7 mM KCl, and 137 mM NaCl; pH 7.5) to 10 µg/mL, and 100 µL of the antibody-containing solution was added to each well of an ELISA plate (manufactured by Thermo Fisher Scientific, Inc., product number 468667). The solution was left to stand overnight under refrigeration, then washed 3 times with 300 µL of TBS-T (50 mM Tris-HCl, 150 mM NaCl, and 0.05% Tween 20; pH 7.5), then 300 µL of TBS containing 1.5% BSA was added, and the mixture was left to stand overnight under refrigeration.

### 3-3. Measurement method

After the TBS containing 1.5% BSA was removed from the prepared antibody-immobilized plate, 100 µL of TBS (50 mM Tris-HCl and 150 mM NaCl, pH 7.5) and 50 µL of the 7 U/L TRACP-5b solution or the 7 U/L TRACP-5a solution were added to each well, and the mixture was reacted for 1 hour under stirring (25°C, 700 rpm). After washing 3 times with 300 µL of TBS-T, 100 µL of a chromogenic substrate solution (5 mM 2-chloro-4-nitrophenyl phosphate, 109 mM MES, and 40 mM sodium tartrate; pH 6.6) to which the evaluation compound was added at a concentration of 0.1 mass% was added to each well, and the plate was left to stand at 37°C for 1 hour. Thereafter, 50 µL of 0.5 N NaOH was added, and the absorbance (primary wavelength: 405 nm, secondary wavelength: 490 nm) was measured with a plate reader.

### 3-4. Measurement results

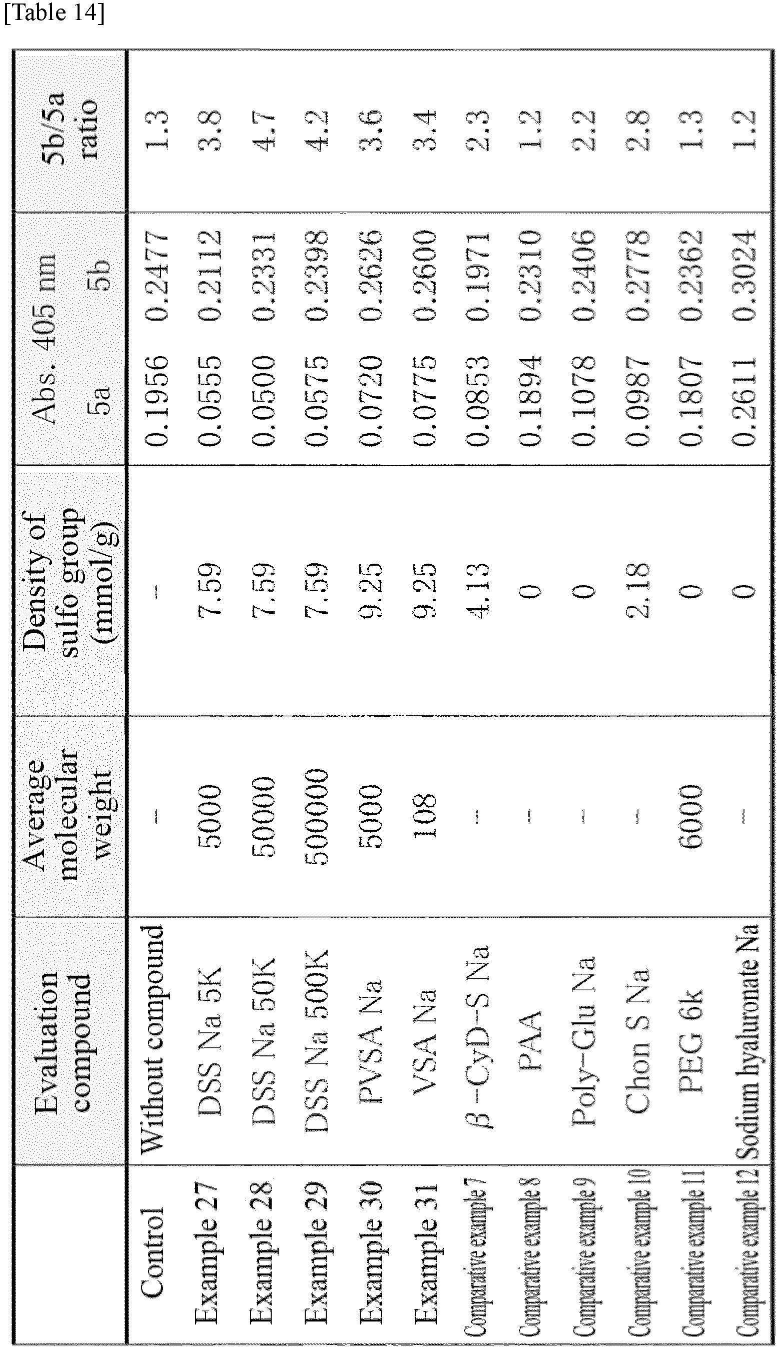

## Claims

1. A method for measuring tartrate-resistant acid phosphatase 5b (TRACP-5b) in a specimen, the method comprising:
performing a reaction for detecting TRACP-5b in the presence of an organic sulfonic acid, sulfate ester, or salt thereof, wherein the organic sulfonic acid, sulfate ester, and/or salt has a sulfo group (-S(O)₂OH), sulfuric acid group (-OS(O)₂OH), and/or salt thereof at a density of 6.5 mmol/g or more.

2. The method according to claim 1, wherein the organic sulfonic acid, sulfate ester, or salt thereof has 50 mass% or more of a sulfo group (-S(O)₂OH), sulfuric acid group (-OS(O)₂OH), and/or salt thereof per unit structure in terms of S(O)₂O⁻.

3. The method according to claim 1 or 2, wherein the organic sulfonic acid, sulfate ester, or salt thereof is represented by Formula (1):
[Chemical Formula 1]
R-Y-S(O)₂OX··· (1)
wherein, R is alkyl, alkenyl, or alkynyl having 1 to 3 carbon atoms, Y is a bond or an oxygen atom, and X is hydrogen or an alkali metal; or
the organic sulfonic acid, sulfate ester, or salt thereof has a repeating unit of Formula (2): 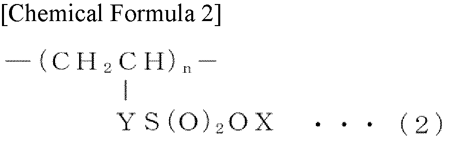
wherein, Y is a bond or an oxygen atom, n is an integer of 2 to 5000, and X is hydrogen or an alkali metal; or
the organic sulfonic acid, sulfate ester, or salt thereof has a repeating unit containing a structure of Formula (3): 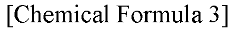 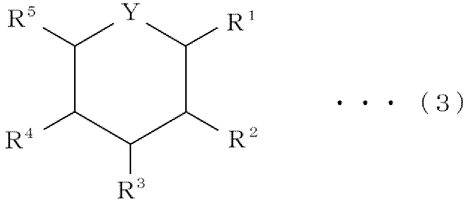
wherein, Y is O or CH₂, two of R¹ to R⁵ are O- or a bond, and at least two of the rest are O-S(O)₂OX or S(O)₂OX in which X is hydrogen or an alkali metal, and those not corresponding to any of the above are OX or C(O)OX in which X is hydrogen or an alkali metal.

4. The method according to claim 1 or 2, wherein the organic sulfonic acid, sulfate ester, or salt thereof is vinyl sulfonic acid or a salt thereof, vinyl sulfate or a salt thereof, polyvinyl sulfonic acid or a salt thereof, polyvinyl sulfuric acid or a salt thereof, methanesulfonic acid or a salt thereof, or dextran sulfate or a salt thereof.

5. The method according to any one of claims 1 to 4, wherein the organic sulfonic acid, sulfate ester, or salt thereof is 1) added independently to a reaction system or a specimen, or contained in 2) a specimen diluent, 3) a substrate-containing reagent, or 4) a reagent containing an anti-TRACP-5b antibody, and thus a reaction for detecting TRACP-5b is performed in the presence of the organic sulfonic acid, sulfate ester, or salt thereof.

6. The method according to any one of claims 1 to 5, wherein measurement is performed by an enzyme method, enzyme immunoassay (EIA), enzyme-linked immunosorbent assay (ELISA), chemiluminescent enzyme immunoassay (CLEIA method), chemiluminescent immunoassay (CLIA method), or latex agglutination method.

7. The method according to any one of claims 1 to 6, wherein the specimen is blood, serum, or plasma.

8. A kit for measuring TRACP-5b, comprising:
an organic sulfonic acid, sulfate ester, and/or salt thereof, wherein the organic sulfonic acid, sulfate ester, and/or salt has a sulfo group (-S(O)₂OH), sulfuric acid group (-OS(O)₂OH), and/or salt thereof at a density of 6.5 mmol/g or more.

9. The kit according to claim 8, wherein the organic sulfonic acid, sulfate ester, and/or salt thereof has 50 mass% or more of a sulfo group (-S(O)₂OH), sulfuric acid group (-OS(O)₂OH), or salt thereof per unit structure in terms of S(O)₂O⁻.

10. The kit according to claim 8 or 9, wherein the organic sulfonic acid, sulfate ester, and/or salt thereof is represented by Formula (1):
[Chemical Formula 4]
R-Y-S(O)₂OX··· (1)
wherein, R is alkyl, alkenyl, or alkynyl having 1 to 5 carbon atoms, Y is a bond or an oxygen atom, and X is hydrogen or an alkali metal; or
the organic sulfonic acid, sulfate ester, and/or salt thereof has a repeating structure of Formula (2): 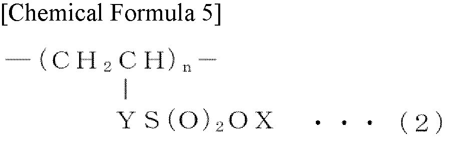
wherein, Y is a bond or an oxygen atom, n is an integer of 2 to 5000, and X is hydrogen or an alkali metal; or
the organic sulfonic acid, sulfate ester, and/or salt thereof has a repeating structure containing a structure of Formula (3): 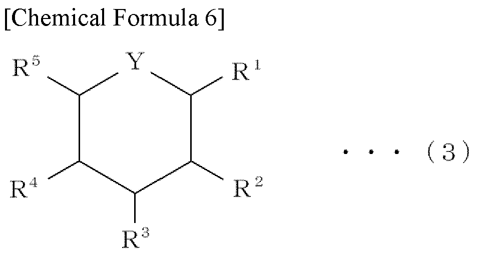
wherein, Y is O or CH₂, two of R¹ to R⁵ are O- or a bond, and at least two of the rest are O-S(O)₂OX or S(O)₂OX in which X is hydrogen or an alkali metal, and those not corresponding to any of the above are OX or C(O)OX in which X is hydrogen or an alkali metal.

11. The kit according to claim 8 or 9, wherein the organic sulfonic acid, sulfate ester, or salt thereof is vinyl sulfonic acid or a salt thereof, vinyl sulfate or a salt thereof, polyvinyl sulfonic acid or a salt thereof, polyvinyl sulfuric acid or a salt thereof, methanesulfonic acid or a salt thereof, or dextran sulfate or a salt thereof.

12. The kit according to any one of claims 8 to 11 for measuring TRACP-5b by an enzyme method, the kit comprising:
a substrate of TRACP-5b; the organic sulfonic acid, sulfate ester, or salt thereof; and optionally a specimen diluent.

13. The kit according to claim 12, wherein the organic sulfonic acid, sulfate ester, or salt thereof is contained in a reagent containing the substrate of TRACP-5b or the specimen diluent.

14. The kit according to any one of claims 8 to 11 for measuring TRACP-5b by enzyme immunoassay (EIA), the kit comprising:
a solid support; an anti-TRACP-5b monoclonal antibody that is adsorbed onto the solid support; a substrate of the TRACP-5b; and the organic sulfonic acid, sulfate ester, or salt thereof; optionally a specimen diluent; and optionally a wash solution.

15. The kit according to claim 14, wherein the organic sulfonic acid, sulfate ester, or salt thereof is contained in a reagent containing the anti-TRACP-5b monoclonal antibody, a reagent containing the substrate of the TRACP-5b, or the specimen diluent.

16. The kit according to any one of claims 8 to 11 for measuring TRACP-5b by enzyme-linked immunosorbent assay (ELISA), the kit comprising:
a solid support; a primary anti-TRACP-5b monoclonal antibody that is adsorbed onto the solid support; a secondary anti-TRACP-5b monoclonal antibody that is capable of binding to TRACP-5b at an epitope different from that of the primary anti-TRACP-5b monoclonal antibody and that is labeled with a labeling substance enabling detection through an enzymatic reaction; a reagent for enabling detection of the labeling substance through an enzymatic reaction; the organic sulfonic acid, sulfate ester, or salt thereof; optionally a specimen diluent; and optionally a wash solution.

17. The kit according to claim 16, wherein the organic sulfonic acid, sulfate ester, or salt thereof is contained in a reagent containing the secondary anti-TRACP-5b monoclonal antibody, the reagent for enabling detection of the labeling substance through an enzymatic reaction, or the specimen diluent.

18. The kit according to any one of claims 8 to 11 for measuring TRACP-5b by chemiluminescent enzyme immunoassay (CLEIA method), the kit comprising:
a solid support; an anti-TRACP-5b monoclonal antibody that is adsorbed onto the solid support; a chemiluminescent substrate of the TRACP-5b; and the organic sulfonic acid, sulfate ester, or salt thereof; optionally a specimen diluent; and optionally a wash solution.

19. The kit according to claim 18, wherein the organic sulfonic acid, sulfate ester, or salt thereof is contained in a reagent containing the chemiluminescent substrate of the TRACP-5b, a reagent containing the anti-TRACP-5b monoclonal antibody, or the specimen diluent.

20. The kit according to any one of claims 8 to 11 for measuring TRACP-5b by chemiluminescent immunoassay (CLIA method), the kit comprising:
a solid support; a primary anti-TRACP-5b monoclonal antibody that is adsorbed onto the solid support; a secondary anti-TRACP-5b monoclonal antibody that is capable of binding to TRACP-5b at an epitope different from that of the primary anti-TRACP-5b monoclonal antibody and that is labeled with a labeling substance enabling detection through chemiluminescence; a reagent enabling detection of the labeling substance through chemiluminescence; the organic sulfonic acid, sulfate ester, or salt thereof; optionally a specimen diluent; and optionally a wash solution.

21. The kit according to claim 20, comprising the organic sulfonic acid, sulfate ester, or salt thereof in a reagent containing the primary anti-TRACP-5b monoclonal antibody, a reagent containing the secondary anti-TRACP-5b monoclonal antibody, a reagent enabling detection of the labeling substance through chemiluminescence, or the specimen diluent.

22. The kit according to any one of 8 to 11 for measuring TRACP-5b by a latex agglutination method, the kit comprising:
latex particles; an anti-TRACP-5b antibody immobilized on the latex particles; the organic sulfonic acid, sulfate ester, or salt thereof; and optionally a specimen diluent.

23. The kit according to claim 22, wherein the organic sulfonic acid, sulfate ester, or salt thereof is contained in a reagent containing the anti-TRACP-5b antibody or the specimen diluent.

24. The kit according to any one of claims 8 to 23, wherein a specimen to be measured is blood, serum, or plasma.
